# EUROPEAN PATENT APPLICATION

(11) **EP 2 457 579 A1**
(43) Date of publication of application: **30.05.2012**
(21) Application number: 10015036.6
(22) Date of filing: 26.11.2010
(51) Int. Cl.: A61K 38/20, C07K 14/54, C12N 15/00

(54) **Covalently linked interleukin -10**

(71) Applicant: Technische Universität Dresden, 01069 Dresden (DE)
(72) Inventor: Roers, Axel, 01326 Dresden (DE); Lanvermann, Sebastian, 50826 Köln (DE); Müller, Werner, 50931 Köln (DE)
(74) Representative: Vossius & Partner

(57) **Abstract**

The present invention relates to a polypeptide having interleukin-10 function, comprising two interleukin-10 monomer subunits covalently linked by a linker. The present invention further relates to a nucleic acid molecule encoding the polypeptide of the invention, a vector comprising said nucleic acid molecule, a non-human host transformed with the nucleic acid molecule or the vector of the invention as well as a method for the production of a recombinant polypeptide of the invention. The present invention further relates to a pharmaceutical composition as well as to the polypeptide, the nucleic acid molecule, the vector or the host or host cell of the invention for use in treating and/or preventing inflammatory diseases.

## Description

The present invention relates to a polypeptide having interleukin-10 function, comprising two interleukin-10 monomer subunits covalently linked by a linker. The present invention further relates to a nucleic acid molecule encoding the polypeptide of the invention; a vector comprising said nucleic acid molecule, a non-human host transformed with the nucleic acid molecule or the vector of the invention as well as a method for the production of a recombinant polypeptide of the invention. The present invention further relates to a pharmaceutical composition as well as to the polypeptide, the nucleic acid molecule, the vector or the host or host cell of the invention for use in treating and/or preventing inflammatory diseases.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.

Invasion of pathogens results in an immune response aiming at efficient pathogen destruction. Several different antimicrobial effector systems of the innate immune system, which are immediately available, represent the first line of defense. Macrophages, granulocytes, mast cells but also many resident non-hematopoietic cell types contribute to innate immunity. These cells sense pathogen invasion by nonspecific recognition of pathogen-associated molecular patterns. Among the pattern recognition receptors, the family of Toll-like receptors (TLRs) has received particular attention. The innate inflammatory response sets the stage for efficient activation of the B and T cell-based adaptive immune system.

While protective on the one hand, immune responses also pose a major threat to the integrity of host tissues, since the immune effectors have the potential to destruct not only microbial but also host cells. An example for immunopathology is endotoxic shock, which results from an overwhelming production of pro-inflammatory mediators in response to LPS exposure and consecutive systemic microvascular damage. Thus, tight regulation of inflammatory responses is important to minimize damage to host tissue in the course of responses to microbial pathogens. Two cytokines pivotal to the control of immune responses are interleukin-10 (IL-10) and transforming growth factor-β (TGF-β).

IL-10 was originally discovered in 1989 as a factor that suppresses the secretion of pro-inflammatory mediators by macrophages *in vitro* (reviewed in Moore, 2001). IL-10 potently limits both innate and adaptive immunity (Moore, 2001). Most hematopoietic cell types but also epithelial cells are capable of secreting IL-10 (Moore, 2001). A number of different T cell subsets have been identified as important regulators of immune responses. The importance of IL-10 secretion by these cells, however, for *in vivo* immune regulation remains unclear. Recently, IL-10 produced by Th1 effector cells was shown to be of critical importance for the control of anti-parasite T cell responses (Anderson, 2007; Jankovic, 2007). Macrophages secrete IL-10 in response to a number of different stimuli and were considered a major source of the cytokine. Compared to pro-inflammatory mediators, which are released early after macrophage activation, IL-10 secretion occurs with a time lag. Macrophage-derived IL-10 may act in an autocrine loop to control macrophage activation (de Waal Malefyt, 1991; Fiorentino, 1991; Kang, 1994; Sutterwala, 1998). IL-10 secretion by B cells was initially thought to be limited to the compartment of B1 cells (O'Garra, 1992). More recently, however, evidence has accumulated that also the B2 cell subset can contribute to the regulation of immune responses by secretion of IL-10 (Fillatreau, 2002; Mangan, 2004; Mauri, 2003; Mizoguchi, 2002).

Humans with a genetic deficiency for IL-10 or the IL-10 receptor develop very severe, intractable inflammatory bowel disease (Glocker, 2009). IL-10 deficient (*IL-10*^{*-*/*-*}) mice mount exaggerated innate immune responses. For example, their sensitivity to bacterial lipopolysaccharide (LPS) is drastically enhanced in comparison to wildtype mice. IL-10^{-/-} mice die of very low amounts of LPS injected intraperitoneally, which are tolerated without significant pathology in wildtype mice (Berg, 1995). After a local subcutaneous injection of LPS, IL-10^{-/-} mice develop extensive neutrophil-dominated inflammatory infiltration and tissue necrosis, while wildtype animals display only mild infiltration with macrophages but no tissue damage (Siewe 2006). IL-10-deficient mice (Kühn, 1993) are also characterized by enhanced Th1 responses to numerous pathogens (Moore, 2001). In many cases, an infection that is cleared without problems in control mice is lethal in IL-10^{-/-} mice due to the uncontrolled T cell-response, which results in severe tissue damage. Depending on the microbial environment, the knock out-animals spontaneously develop inflammatory bowel disease (Kühn 1993).

Asadullah *et al.* 2003 describes various attempts to treat several inflammatory disorders in humans by systemic administration of recombinant IL-10. As discussed by the authors, systemic administration of IL-10 so far has mainly provided unsatisfactory results in diseases such as Crohn's disease or rheumatoid arthritis. The authors conclude that applying IL-10 locally in high concentrations might be a more promising approach than systemic application.

Thus, despite the potent anti-inflammatory IL-10 effects observed *in vitro,* there remains a need to provide cytokines having improved properties *in vivo.*

This need is addressed by the provision of the embodiments characterised in the claims.

Accordingly, the present invention relates to a polypeptide having cytokine function, comprising two cytokine monomer subunits covalently linked by a linker.

The term "polypeptide" as used herein interchangeably with the term "protein" describes linear molecular chains of amino acids, including single chain proteins or their fragments, containing more than 30 amino acids. Accordingly, the term "peptide" as used in the present invention describes linear chains of amino acids containing up to 30 amino acids. Polypeptides may further form oligomers consisting of at least two identical or different molecules. The corresponding higher order structures of such multimers are, correspondingly, termed homo- or heterodimers, homo- or heterotrimers etc. Homodimers etc. of cytokine, such as IL-10, monomer subunits giving rise to the corresponding polypeptide having cytokine (e.g. IL-10) function of the present invention thus fall under the definition of the term "polypeptide". Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The term "polypeptide" also refers to naturally modified polypeptides where the modification is effected e.g. by glycosylation, acetylation, phosphorylation and similar modifications which are well known in the art.

The term "cytokine", in accordance with the present invention, relates to small cell-signaling proteins that act as immuno-modulating agents. Cytokines include for example interleukins, interferons, granulocyte-macrophage colony-stimulating factor and chemokines and are secreted by numerous cells, such as e.g. glial cells of the nervous system and cells of the immune system. The term "having cytokine function", as used herein, refers to the below described functions of the respective cytokines, that are well known to the person skilled in the art. In other words, where the polypeptide having cytokine function comprises e.g. two IFN monomer subunits covalently linked by a linker, the cytokine function is the below described function of interferon. Means and methods for testing whether a polypeptide has the desired function are well known in the art.

Interleukins (ILs) are mainly synthesized by helper CD4+ T lymphocytes, but also by through monocytes, macrophages, and endothelial cells. Interleukins include IL-1, IL-2, IL-3, IL-4, IL-5, IL-6, IL-7, IL-8, IL-9, IL-10, IL-11, IL-12, IL-13, IL-14, IL-15, IL-16, IL-17, IL-18, IL-19, IL-20, IL-21, IL-22, IL-23, IL-24, IL-25, IL-26, IL-27, IL-28, IL-29, IL-30, IL-31, IL-32, IL-33 and IL-35. Their functions include, without being limiting, stimulation of growth and differentiation of T cell response, activation of NK cells, maturation and proliferation of B-cells, co-stimulation of T helper cells, differentiation and proliferation of myeloid progenitor cells, growth promotion of mast cells and histamine release therefrom, proliferation and differentiation of activated B cells as well as IgG1 and IgE synthesis, production of eosinophils or antibody secretion from plasma cells.

Interferons (IFNs) are made and released by lymphocytes in response to the presence of tumor cells or pathogens such as e.g. viruses, bacteria, or parasites. IFNs enable communication between cells, which then triggers the protective defenses of the immune system that attack pathogens or tumors. IFNs further activate immune cells, such as natural killer cells and macrophages, increase the recognition of infections or tumor cells by up-regulating antigen presentation to T lymphocytes and increase the ability of uninfected host cells to resist new infection by virus. So far, the interferons IFN-α, IFN-β, IFN-ε, -κ, -τ, -δ, and -ζ, IFN-ω, IFN-ν (type I interferons), IFN-γ (type II interferon) and IFN-λ1, IFN-λ2 and IFN-λ3 (type III interferon) have been identified in mammals.

Granulocyte-macrophage colony-stimulating factor, also referred to as GM-CSF, is a cytokine secreted by macrophages, T cells, mast cells, endothelial cells and fibroblasts. GM-CSF functions as a white blood cell growth factor. It stimulates stem cells to produce granulocytes (neutrophils, eosinophils, and basophils) and monocytes. Monocytes exit the circulation and migrate into tissue, whereupon they mature into macrophages. It is thus part of the immune/inflammatory cascade, by which activation of a small number of macrophages can rapidly lead to an increase in their numbers, a process crucial for fighting infection.

Chemokines are cytokines that have the ability to induce directed chemotaxis in nearby responsive cells - i.e. they are chemotactic cytokines. Chemokines have shared structural characteristics such as small size (approximately 8-10 kilodaltons in size), and the presence of four cysteine residues in conserved locations that are key to forming their 3-dimensional shape. Chemokines function as chemoattractant to guide the migration of cells, which follow a signal of increasing chemokine concentration towards the source of the chemokine. Some chemokines control cells of the immune system during processes of immune surveillance, such as directing lymphocytes to the lymph nodes enabling them to screen for invasion of pathogens by interacting with antigen-presenting cells residing in these tissues. Chemokines can also act inflammatory and are released from a wide variety of cells in response to bacterial infection, viruses and agents that cause physical damage such as silica or the urate crystals that occur in gout. Inflammatory chemokines function mainly as chemoattractants for leukocytes, recruiting monocytes, neutrophils and other effector cells from the blood to sites of infection or tissue damage.

Chemokines include the group of CC chemokines (or ß-chemokines), including at least 27 members, the group of CXC chemokines (or α-chemokines), including at least 17 different members, the group of C chemokines (or γ chemokines), for which only two members have been described so far and the group of CX₃C chemokines (or d-chemokines), for which only fractalkine (or CX₃CL1) has been discovered so far.

Preferably, the cytokine is selected from the group of cytokines that forms dimers or that act as dimers upon binding to their respective receptors. More preferably, the cytokine is selected from the group consisting of IL-5, INFγ and IL-6. The amino acid sequences for IL-5, INFγ and IL-6 are well known and have been published in protein databases. These sequences include, for example, human and mouse IL-5 (NP_000870.1 and NP_034688.1; as shown in SEQ ID NOs: 11 and 12), human and mouse INFγ (AAB59534.1 and AAI19064.1; as shown in SEQ ID NOs: 13 and 14) and human and mouse IL-6 (NP_000591.1 and NP_112445.1; as shown in SEQ ID NOs: 15 and 16). It will be appreciated by the skilled person that the polypeptide of the invention will comprise monomers of the mature polypeptide. The term "linker" is well known in the art and relates to means for attaching a first moiety to a second moiety by introduction of a third moiety which is referred to as linker. Said attaching involves two covalent bonds, one between the linker and the first moiety and a second between the linker and the second moiety. In accordance with the present invention, the term "linker" relates to peptide linkers as well as to non-peptide linkers, which covalently link the cytokine monomer subunits, such as e.g. IL-10 monomer subunits.

"Peptide linkers" as envisaged by the present invention are linkers of at least 1 amino acid in length. Preferably, the linker has a length of between at least 1 amino acid and less than 100 amino acids, such as for example between at least 2 amino acids and less than 75 amino acids, more preferably between at least 3 amino acids and less than 50 amino acids, such as for example between at least 4 amino acids and less than 25 amino acids, such as for example between at least 5 amino acids and less than 20 amino acids and even more preferably between at least 6 amino acids and less than 15 amino acids. More preferably, the linker has a length of between at least 7 amino acids and less than 10 amino acids.

Most preferably, the linker has a length of 7 amino acids.

In a preferred embodiment, the linker is a flexible linker. Preferably, the flexible linker comprises or consists of the amino acids glycine, aspargine and/or serine. More preferably, the flexible linker comprises or consists of the amino acids glycine and serine.

It will be appreciated by the skilled person that when the polypeptide of the invention is a single polypeptide chain, the linker is a peptide linker. Alternatively, when the polypeptide of the invention comprises two or more separate polypeptide chains, the linker may also be a non-peptide linker.

The term "non-peptide linker", as used in accordance with the present invention, refers to linkage groups having two or more reactive groups but excluding peptide linkers as defined above. For example, the non-peptide linker may be a polymer having reactive groups at both ends. The reactive groups of the non-peptide linker individually bind to reactive groups of the polypeptide of the invention, for example, to a lysine residue, a histidine residue or a cysteine residue. The reactive groups of the non-peptide linker include an aldehyde group, a propionic aldehyde group, a butyl aldehyde group, a maleimide group, a ketone group, a vinyl sulfone group, a thiol group, a hydrazide group, a carbonyldimidazole (CDI) group, a nitrophenyl carbonate (NPC) group, a trysylate group, an isocyanate group, and succinimide derivatives. Examples of succinimide derivatives include succinimidyl propionate (SPA), succinimidyl butanoic acid (SBA), succinimidyl carboxymethylate (SCM), succinimidyl succinamide (SSA), succinimidyl succinate (SS), succinimidyl carbonate, and N-hydroxy succinimide (NHS). The reactive groups at both ends of the non-peptide linker may be the same or different. For example, the non-peptide linker may have a maleimide group at one end and an aldehyde group at another end.

The linker serves to keep the two monomers in close contact, thus preventing dissociation of the polypeptide of the invention. At the same time, the linker serves to physically separate the monomers in the polypeptide of the invention, thus ensuring that the monomers are sufficiently distanced from each other to enable dimerisation of the monomers in an anti-parallel orientation. Thus, preferred linkers should not interfere with the formation of a homodimer, as it is a prerequisite that the polypeptide of the invention comprising monomers covalently linked by a linker maintains its biological function as defined above, such as e.g. the capacity to interact and activate the respective cytokine receptor (e.g. the IL-10 receptor). It will be understood by one of skill in the art that while a linker may be employed that covalently links the two monomers via their C-termini, such a linker has to be sufficiently long and/or flexible to still enable the formation of a dimer having an anti-parallel orientation of the two monomers. Furthermore, preferred linkers should adopt a flexible conformation and should have minimal hydrophobic or charged character, to avoid interaction with the functional protein domains and/or solvent. Preferably, the linker is chosen to be a moiety capable of avoiding detection by the immune system. The skilled person knows how to design appropriate linker molecules based on his/her common knowledge. For example, peptide linkers can be chosen from the LIP (Loops in Proteins) database (Michalsky et al., 2003) obtained commercially (see, for example, the catalogue from Glen Research, 22825 Davis Drive, Sterling, Virginia, 20164 USA).

The linker may be appended to the N- or the C-terminus or, if deemed suitable, also to an amino acid apart from the terminal amino acids of the polypeptide of the present invention. The linker preferably connects the C-terminus of one monomer and the N-terminus of the other monomer.

The skilled person is well aware of methods to test the suitability of different linkers. For example, the properties of the linker can easily be tested by comparing the activity of the polypeptide having a certain cytokine function of the present invention with the respective naturally occurring, non-covalently linked cytokine. Similarly, the activity of a polypeptide having IL-10 function of the present invention can be compared with naturally occurring, non-covalently linked IL-10, as described for example in the appended examples. The stability of the resulting molecule can also be measured by e.g. melting point analysis in circular dichroism spectroscopy.

In an alternative embodiment, or in a preferred embodiment, the present invention relates to a polypeptide having interleukin-10 function, comprising two interleukin-10 monomer subunits covalently linked by a linker.

The term "interleukin-10", as used in accordance with the present invention, relates to an anti-inflammatory cytokine capable of inhibiting synthesis of pro-inflammatory cytokines such as for example IFN-γ, IL-2, IL-3, TNFα and GM-CSF. Interleukin-10 is also referred to herein as IL-10. Naturally occurring interleukin-10 is a homodimer of two identical polypeptide chains non-covalently associated in an anti-parallel orientation (Moore 2001). In humans, each monomer is approx. 18 kD and is composed of 178 amino acids, of which an 18 amino acid signal peptide is cleaved off during maturation of the monomer, thus resulting in a 160 amino acid monomer. The sequence of IL-10 has been elucidated for a large amount of different species (reviewed in Moore 2001). For example, the Genbank database contains entries for the human (Vieira et al., 1991), mouse (Moore et al., 1990), rat (Langley et al., 2001), guinea pig (Scarozza et al., 1998), dog (Lu et al., 1995), monkey (Villinger et al., 1995) cow or pig (Blancho et al., 1995) interleukin-10 monomers. Furthermore, viral IL-10 homologs with striking similarity to host IL-10 proteins have been identified, for example in Epstein-Barr virus (BCRF1) (Hsu et al., 1990), herpes virus type 2 (Rode et al., 1994), cytomegalovirus (Kotenko et al., 2000; Spencer, 2002), and Orf virus (Fleming et al., 1997).

Naturally occurring IL-10 is encoded by the IL-10 gene, which is highly conserved between species, with e.g. mouse and human IL-10 being 73% identical. In humans, the IL-10 gene is located in chromosome 1 and consists of 5 exons. The protein is characterized by an α-helical bundle structure similar to interferons and hematopoietic cytokines (Moore, 2001). The receptor for IL-10 is composed of at least two subunits that are members of the interferon receptor family: IL-10R1 is the ligand-binding subunit and IL-10R2 represents an accessory subunit for signaling (Moore 2001).

Human IL-10 is believed to not be glycosylated, while both recombinant and T cell-derived murine IL-10 appear to be heterogeneously N-glycosylated at a site near the N-terminus. Glycosylation of murine IL-10 does not appear to have any influence on biological activity. Human IL-10 is active on both mouse and human cells, whereas murine IL-10 is effective only on mouse cells. Biochemical (Syto et al. 1995) and X-ray crystallographic analyses (Walter 1998) of human IL-10 demonstrated that IL-10 is an acid-sensitive, non-covalent homodimer of two interpenetrating polypeptide chains, similar to interferon-γ. Syto *et al.* demonstrated that around pH 5.5 - which is also measured on sites of inflammation - human IL-10 significantly looses its potential to induce proliferation of an IL-10-depependent cell line and further that this loss of function was due to dimer dissociation.

The term "interleukin-10 function", as used herein, denotes in particular any known biological function or activity of interleukin-10. Examples of said biological function or activity include its anti-inflammatory activities, such as the inhibition of the synthesis of a number of cytokines, including IFN-gamma, IL-2, IL-3, TNF and GM-CSF produced by activated macrophages and by helper T-cells. Further examples are the down-regulation of the expression of MHC class II antigens and co-stimulatory molecules on antigen presenting cells. It also includes the enhancing of B cell survival, proliferation, and antibody production. Furthermore, IL-10 can block NF-κB activity. IL-10 signals via the JAK-STAT signaling pathway.

All these functions or activities can be tested for either using any of a variety of standard methods known in the art, such as e.g. measuring the induction of Stat3-phosphorylation or the suppression of LPS-induced release of TNFα, induction of proliferation of IL-10-dependent cell lines as shown in the examples below or on the basis of the teachings of the documents cited therein.

As used in accordance with the present invention, the term "monomer subunits" relates to IL-10 polypeptide chains of approx. 18 kDa, as described above. Naturally occurring IL-10 is a homodimer of two such polypeptide chains that are non-covalently associated.

In accordance with the present invention, it was shown that a polypeptide containing two IL-10 monomers covalently connected by a linker is folded correctly and displays biological activity *in vitro* and *in vivo.* As shown in the examples below, bioactivity was determined in three different assays (induction of STAT3 phosphorylation in cultivated murine splenocytes, induction of proliferation of the IL-10 dependent cell line Ba/F3, suppression of LPS-induced TNFα release from murine splenocytes in vitro). In these assays, two preparations of commercial bacterially expressed murine IL-10 did not display any biological activity while the polypeptide having IL-10 function according to the present invention expressed in human cells was active. A comparison of the wildtype IL-10 and the polypeptide having IL-10 function of the invention (both expressed in parallel in human cells and treated equally) in these *in vitro* assays showed an activity of the polypeptide of the invention at least equal to the activity of non-covalently linked murine wildtype IL-10.

It was previously shown in mouse lines with a selective inactivation of the IL-10 gene in particular cell types, such as B cells, T cells, macrophages or mast cells, that IL-10 secretion by different cellular sources can serve distinct and non-redundant functions. In other words, it was found that the cellular source critically determines the biological effect of this cytokine (Roers 2004, Siewe 2006, Rubtsov 2008, Igyarto 2009). These cell type-specific effects seem to imply that an IL-10-responsive cell can discriminate between different cellular sources of the cytokine. Without wishing to be bound by any particular theory, it is hypothesized by the inventors of the present invention that such a specificity for a particular source may result if IL-10 action is short-ranged only and limited to the immediate vicinity of the IL-10-secreting cell. A molecular mechanism to ensure such a short-ranged action could be the rapid dissociation of the IL-10 dimer into less active monomers, in particular at conditions of low pH, which are encountered at sites of inflammation.

As is shown in example 5, co-injection of murine IL-10 together with LPS into the skin of IL-10^{-/-} mice results in efficient suppression of tissue inflammation. However, a rapid dissociation into less active or inactive monomers might explain the lack of success when attempting to treat various diseases by systemic administration of IL-10. The polypeptide having IL-10 function of the present invention overcomes these drawbacks and offers a novel tool for the suppression of inflammation *in vivo* both after local and/or systemic administration, as it is significantly more stable at sites of inflammation as compared to wildtype IL-10 and, therefore, more active. Thus, the polypeptide of the present invention represents a new anti-inflammatory compound that is more effective in the treatment of inflammatory diseases than wildtype IL-10.

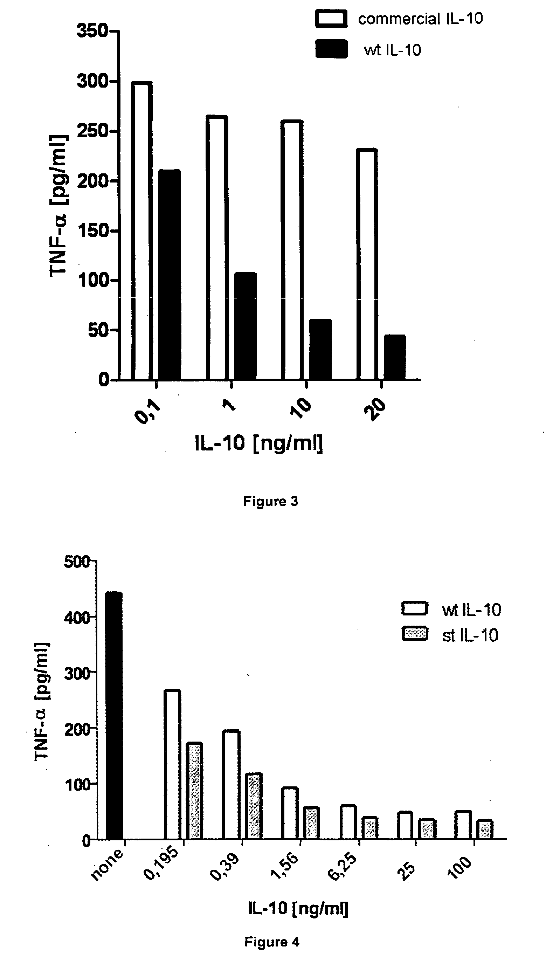

Furthermore, the polypeptide of the present invention simplifies the production of the respective cytokine, such as e.g. IL-10 for experimental or therapeutic use. As discussed above, commercial murine IL-10 obtained from two different manufacturers did not display any biological activity (see e.g. fig. 3), while the IL-10 prepared in accordance with the present invention reliably showed robust activity in a number of bioassays. This observation may be, as discussed above, due to limited stability of the wildtype homo-dimer. Furthermore, it is expected that the intra-molecular dimer-formation of the polypeptide of the present invention may occur more readily as compared to dimerisation after *in vitro* expression of the wildtype cytokine, due to the close physical association of the monomers.

Finally, the polypeptide of the present invention also provides the opportunity to develop new biomaterials. An emerging new field is the design of artificial matrices or scaffolds that are loaded with growth factors or cytokines to facilitate processes like tissue regeneration or wound healing (Hubbell 2005). For example, IL-10 has important effects on tissue repair (Eming 2007). Excisional wounds in IL-10-deficient mice display a more intense inflammatory tissue response, heal faster but result in enhanced collagen-deposition and thus increased scar tissue. In several areas of modern medicine, e.g. in ophthalmic or otolaryngeal microsurgery, including cosmetic surgery, minimal scarring is of prime importance. Reduction of scarring by wound dressing composed of intelligent artificial matrices or scaffolds loaded with IL-10 protein could be beneficial in these situations. This approach requires covalent coupling of IL-10 (or other beneficial cytokines) to artificial surfaces, which proves difficult for the anti-parallel wildtype homo-dimer, which requires multiple coupling groups to enable the formation of, or maintenance of, dimers. On the other hand, covalent coupling of e.g. IL-10 to artificial surfaces will be greatly facilitated by the use of the polypeptide of the invention, as only one coupling group will be required. Thus, problems of sterical hindrance can be expected to be less significant compared to a situation where both monomers are coupled to a surface. Furthermore, the increased stability of the linked dimer is expected to result in higher biological activity as compared to artificial biomaterials loaded with wildtype cytokines, such as IL-10.

In a preferred embodiment of the polypeptide of the invention, the interleukin-10 monomers are independently selected from the group consisting of mammalian- and virus-derived interleukin-10 monomers.

More preferably, the mammalian-derived interleukin-10 monomers are selected from the group consisting of human (Vieira et al., 1991), mouse (Moore et al., 1990), rat (Langley et al., 2001), guinea pig (Scarozza et al., 1998), dog (Lu et al., 1995), monkey (Villinger et al., 1995) cow or pig (Blancho et al., 1995) interleukin-10 monomers. Also preferred is that the virus-derived interleukin-10 monomers are selected from the group consisting of Epstein-Barr virus (BCRF1) (Hsu et al., 1990), herpes virus type 2 (Rode et al., 1994), cytomegalovirus (Kotenko et al., 2000; Spencer, 2002), and Orf virus (Fleming et al., 1997).

The term "independently selected", as used herein, encompasses the selection of monomers from the same species but also the selection of monomers from different species, such as for example one monomer selected from human and one monomer selection from mouse.

In a more preferred embodiment, the interleukin-10 monomers are independently selected from (i) any one of SEQ ID NOs:1 to 7, and/or (ii) a sequence having at least 70% sequence identity to SEQ ID NO:1 and essentially retaining the biological activity of IL-10.

SEQ ID NO:1 corresponds to the mature human IL-10 monomere (based on RefSeq accession number NP_000563.1, without the signalling peptide), while SEQ ID NO:2 corresponds to mature murine IL-10 monomere (based on RefSeq accession number NP_034678.1, without the signalling peptide), SEQ ID NO:3 corresponds to the mature monkey monomere (based on RefSeq accession number NP_001038192.1, without the signalling peptide), SEQ ID NO:4 corresponds to the dog monomere (RefSeq accession number 001003077.1), SEQ ID NO:5 corresponds to the rat monomere (RefSeq accession number NP_036986), SEQ ID NO:6 corresponds to the guinea pig monomere (UniProt accession number Q9Z1Y5) and SEQ ID NO:7 corresponds to the swine monomere (RefSeq accession number NP_999206.1). It will be appreciated by the skilled person that also the immature polypeptides may be employed in order to express the respective monomers of human, mouse or monkey IL-10 in a cell prior to purification and covalent linkage thereof. Such immature forms of IL-10 monomers will be processed by the host or host cell by cleavage of the signaling peptide or may be cleaved *in vitro* after purification and before linkage. In that case, the monomers may be selected from SEQ ID NOs: 8 to 10, which represent human, mouse or monkey IL-10 monomers with the signaling peptide, respectively.

In accordance with this embodiment, the IL-10 monomers may be selected from a sequence having at least 70% sequence identity to SEQ ID NO:1. More preferably, the IL-10 monomers are selected from a sequence that has at least 80%, even more preferably at least 85% sequence identity to SEQ ID NO:1. Even more preferably the IL-10 monomers are selected from a sequence that has at least 90 % sequence identity to SEQ ID NO:1, such as at least 95% sequence identity and most preferably at least 98% sequence identity to SEQ ID NO:1. Such molecules may be splice forms, homologous molecules from other species, such as orthologs, or mutated sequences from the same species to mention the most prominent examples.

In accordance with the present invention, the term "% sequence identity" describes the number of matches ("hits") of identical amino acids of two or more aligned amino acid sequences as compared to the number of amino acid residues making up the overall length of the amino acid sequences (or the overall compared part thereof). In other terms, using an alignment, for two or more sequences or sub-sequences the percentage of amino acid residues that are the same (e.g., 80% or 85% identity) may be determined, when the (sub)sequences are compared and aligned for maximum correspondence over a window of comparison, or over a designated region as measured using a sequence comparison algorithm as known in the art, or when manually aligned and visually inspected. Preferred polypeptides in accordance with the invention are those where the described identity exists over a region that is at least about 15 to 25 amino acids in length, more preferably, over a region that is at least about 50 to 100 amino acids in length. More preferred polypeptides in accordance with the present invention are those having the described sequence identity over the entire length of the polypeptide. Those having skill in the art will know how to determine percent sequence identity between/among sequences using, for example, algorithms such as those based on the NCBI BLAST algorithm (Stephen F. Altschul, Thomas L. Madden, Alejandro A. Schäffer, Jinghui Zhang, Zheng Zhang, Webb Miller, and David J. Lipman (1997), "Gapped BLAST and PSI-BLAST: a new generation of protein database search programs", Nucleic Acids Res. 25:3389-3402), CLUSTALW computer program (Thompson Nucl. Acids Res. 2 (1994), 4673-4680) or FASTA (Pearson and Lipman, Proc. Natl. Acad. Sci., 1988, 85; 2444), as known in the art. The NCBI BLAST algorithm is preferably employed in accordance with this invention. For amino acid sequences, the BLASTP program uses as default a word length (W) of 3, and an expectation (E) of 10. The BLOSUM62 scoring matrix (Henikoff, Proc. Natl. Acad. Sci., 1989, 89:10915) uses alignments (B) of 50, expectation (E) of 10, M=5, N=4, and a comparison of both strands. Accordingly, all the polypeptides having the prescribed function and further having a sequence identity of at least 70% as determined with the NCBI BLAST program fall under the scope of the invention.

In accordance with the present invention, the biological activity of IL-10 is essentially retained if at least 60% of the biological activity of IL-10 is retained. Preferably, at least 75% or more preferably at least 80% of the IL-10 activity is retained. More preferred is that at least 90% such as at least 95%, even more preferred at least 98% such as at least 99% of the biological activity of IL-10 is retained. Most preferred is that the biological activity is fully, i.e. to 100%, retained. Also in accordance with the invention are polypeptides having increased biological activity compared to IL-10, i.e. more than 100% activity. It will be understood by the person skilled in the art that the biological activity of IL-10 refers to the IL-10 function defined herein above. In accordance with this preferred embodiment, the requirement that the interleukin-10 monomers essentially retain the biological activity of IL-10 refers to the biological activity of the IL-10 molecule resulting from the dimerisation of the respective IL-10 monomers. In other words, the monomers have to retain the capability to form functional IL-10 dimers having the above defined minimal biological activity as compared to naturally occurring IL-10. Methods of assessing biological activity of an IL-10 polypeptide have been discussed herein above.

In a further more preferred embodiment of the polypeptide of the invention, the interleukin-10 monomers are independently selected from SEQ ID NO:1 or SEQ ID NO:2.

In an even more preferred embodiment, the two IL-10 monomers are identical in their amino acid sequence.

In another preferred embodiment of the polypeptide of the invention, the linker is a peptide linker.

In a further preferred embodiment of the polypeptide of the invention, the polypeptide is a single polypeptide chain.

In a more preferred embodiment of the polypeptide of the invention, the peptide linker is selected from the group consisting of (Gly₃SerGly₃) ₙ, (Gly₂Ser₁Gly₂) ₙ, (GlySer₁Gly) ₙ, and (Gly₃Ser₁Gly₂)ₙ, wherein n is an integer independently selected from 0, 1, 2, 3 or 4.

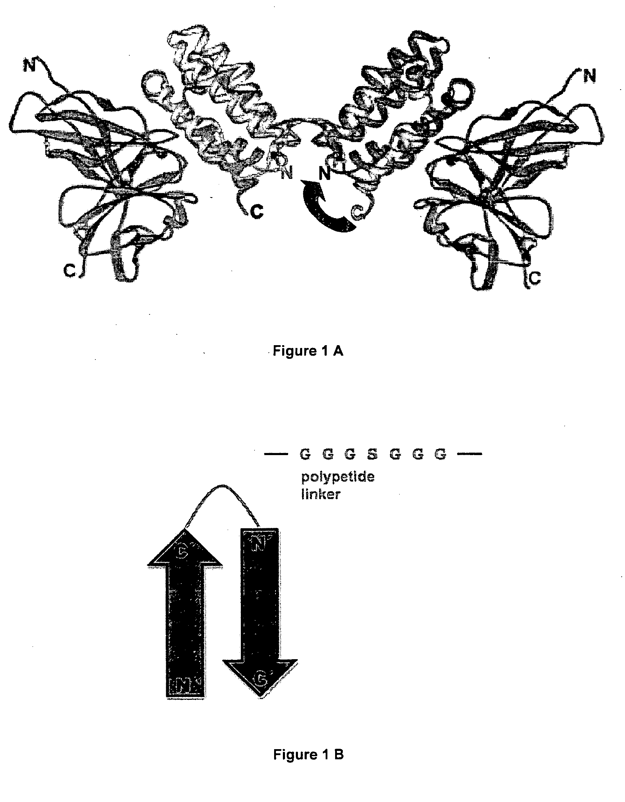

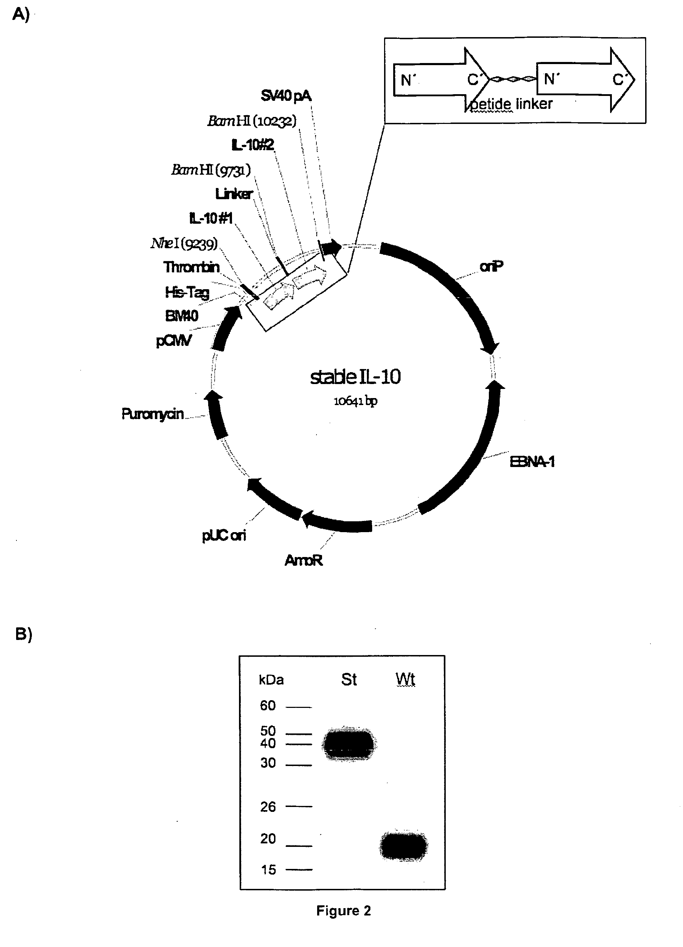

Preferably the linker sequence is Gly₃SerGly₃.
the resulting dimeric IL-10 protein is enhanced, such that the N-terminus of one subunit is located adjacent to the C-terminus of the other subunit and vice-versa. The anti-parallel orientation of the resulting IL-10 dimer is shown in figures 1 and 2.

In a further preferred embodiment of the polypeptide of the present invention, the polypeptide further comprises at least one additional polypeptide or peptide.

Preferably, the additional polypeptide or peptide (referred to herein as (poly)peptide) is unrelated to cytokines and can be, for example, a tag or a functional (poly)peptide suitable to improve the performance of the polypeptide of the invention. The tag can e.g. be a Strep-tag, a His-tag, a Myc-tag or a Flag-tag. Functional (poly)peptides are e.g. a kappa secretion leader, human serum albumin (hsa) or fragments thereof, (poly)peptides capable of binding to hsa or other serum proteins; (poly)peptides capable of binding to neonatal Fc receptor (FcRn), human muscle aldolase (hma) or fragments thereof, CD8 hinge region, immunoglobulin constant regions or variable regions.

The term "fragments thereof" in connection with the present invention refers to fragments of the molecules still having one or more of the biological functions of the full-length molecules. It is well known in the art that functional molecules, such as for examples (poly)peptides may be cleaved to yield fragments with unaltered or substantially unaltered function. Such cleavage may include the removal of a given number of N- and/or C-terminal amino acids. Additionally or alternatively, a number of internal (non-terminal) amino acids may be removed, provided the obtained (poly)peptide has the function of the full length (poly)peptide. Said number of amino acids to be removed from the termini and/or internal regions may be one, two, three, four, five, six, seven, eight, nine, ten, 15, 20, 25, 30, 40, 50 or more than 50. Any other number between one and 50 is also deliberately envisaged in accordance with this invention.

Means and methods for determining such functional domains of (poly)peptides are well known in the art and include experimental and bioinformatic means. Experimental means include the systematic generation of deletion mutants and their assessment in assays for the desired functions above known in the art. Bioinformatic means include database searches. Suitable databases included protein sequence databases as well as databases for glycobiology. In this case a multiple sequence alignment of significant hits is indicative of domain boundaries, wherein the domain(s) is/are comprised of the/those sub-sequences exhibiting an elevated level of sequence conservation as compared to the remainder of the sequence. Further suitable databases include databases of statistical models of conserved protein domains such as Pfam maintained by the Sanger Institute, UK (www.sanger.ac.uk/Software/Pfam).

In particular, the additional (poly)peptides or the fragments of (poly)peptides as envisaged in this embodiment are capable of increasing the stability and/or the serum half-life of the polypeptide of the present invention. In addition, the use of immunoglobulin variable regions as additional (poly)peptides or fragments thereof enables the generation of polypeptides in accordance with the present invention that can be targeted to a specific cell type. Such a polypeptide would specifically act on this particular cell type. Furthermore, the additional (poly)peptides or fragments thereof may facilitate the purification of the polypeptide of the invention when recombinantly expressed. Finally, the polypeptide of the invention could thus be introduced into bacteriophages as part of the bacteriophage proteins and variants of the polypeptide of the invention with new binding properties could be select by phage binding assays.

Methods to add tags and/or other (poly)peptides to the polypeptide of the present invention are well known to the skilled person and described e.g. in Sambrook, 2001, loc. cit.

The present invention further relates to a nucleic acid molecule encoding the polypeptide of the present invention.

In accordance with the present invention the term "nucleic acid molecule" defines a linear molecular chain consisting of more than 100 nucleotides. The group of molecules designated herein as "nucleic acid molecules" also comprises complete genes.

"Nucleic acid molecules", in accordance with the present invention, include DNA, such as for example cDNA or genomic DNA, and RNA, for example mRNA. Further included are nucleic acid mimicking molecules known in the art such as for example synthetic or semi-synthetic derivatives of DNA or RNA and mixed polymers. Such nucleic acid mimicking molecules or nucleic acid derivatives according to the invention include phosphorothioate nucleic acid, phosphoramidate nucleic acid, 2'-O-methoxyethyl ribonucleic acid, morpholino nucleic acid, hexitol nucleic acid (HNA) and locked nucleic acid (LNA) (see Braasch and Corey, Chem Biol 2001, 8:1). LNA is an RNA derivative in which the ribose ring is constrained by a methylene linkage between the 2'-oxygen and the 4'-carbon. They may contain additional non-natural or derivative nucleotide bases, as will be readily appreciated by those skilled in the art.

It will be appreciated by the skilled person that the nucleic acid molecule of the invention encodes the polypeptide of the invention in those instances where the polypeptide is a single-chain polypeptide, i.e. when the linker is a peptide linker.

The present invention also relates to a vector comprising the nucleic acid molecule of the invention.

Preferably, the vector is a plasmid, cosmid, virus, bacteriophage or another vector used e.g. conventionally in genetic engineering.

The nucleic acid molecule of the present invention may be inserted into several commercially available vectors. Non-limiting examples include prokaryotic plasmid vectors, such as the pUC-series, pBluescript (Stratagene), the pET-series of expression vectors (Novagen) or pCRTOPO (Invitrogen) and vectors compatible with expression in mammalian cells like pCEP4 (Invitrogen), pREP (Invitrogen), pSecTag2HygroC (Invitrogen), pcDNA3 (Invitrogen), pMC1neo (Stratagene), pXT1 (Stratagene), pSG5 (Stratagene), EBO-pSV2neo, pBPV-1, pdBPVMMTneo, pRSVgpt, pRSVneo, pSV2-dhfr, pIZD35, pLXIN, pSIR (Clontech), pIRES-EGFP (Clontech), pEAK-10 (Edge Biosystems) pTriEx-Hygro (Novagen) and pCINeo (Promega). Examples for plasmid vectors suitable for Pichia pastoris comprise e.g. the plasmids pAO815, pPIC9K and pPIC3.5K (all Intvitrogen).

The nucleic acid molecule of the present invention may also be inserted into vectors such that a translational fusion with another nucleic acid molecule is generated. The other nucleic acid molecules may encode a (poly)peptide which can e.g. increase the solubility and/or facilitate the purification of the protein encoded by the nucleic acid molecule of the invention. Non-limiting examples include pET32, pET41, pET43 (Novagen). The vectors may also contain an additional expressible polynucleotide coding for one or more chaperones to facilitate correct protein folding. Suitable bacterial expression hosts comprise e. g. strains derived from BL21 (such as BL21(DE3), BL21(DE3)PlysS, BL21(DE3)RIL, BL21(DE3)PRARE) or Rosetta®.

For vector modification techniques, see Sambrook and Russel "Molecular Cloning, A Laboratory Manual", Cold Spring Harbor Laboratory, N.Y. (2001). Generally, vectors can contain one or more origins of replication (ori) and inheritance systems for cloning or expression, one or more markers for selection in the host, e. g., antibiotic resistance, and one or more expression cassettes. Suitable origins of replication (ori) include, for example, the Col E1, the SV40 viral and the M 13 origins of replication.

A typical mammalian expression vector contains the promoter element, which mediates the initiation of transcription of mRNA, the protein coding sequence, and signals required for the termination of transcription and polyadenylation of the transcript. Moreover, elements such as origin of replication, drug resistance gene, regulators (as part of an inducible promoter) may also be included. The *lac* promoter is a typical inducible promoter, useful for prokaryotic cells, which can be induced using the lactose analogue *isopropylthiol-b-D-galactoside.* ("IPTG"). Preferably, the nucleic acid molecule of the invention is operably linked to such expression control sequences allowing expression in prokaryotes or eukaryotic cells. The vector may further comprise nucleotide sequences encoding secretion signals as further regulatory elements. Such sequences are well known to the person skilled in the art. This sequence is typically located immediately 5' to the gene encoding the polypeptide of the invention, and will thus be transcribed at the amino terminus thereof. However, in certain cases, the signal sequence has been demonstrated to be located at positions other than 5' to the gene encoding the protein to be secreted. This sequence targets the protein to which it is attached across the inner membrane of e.g. a bacterial cell. The DNA encoding the signal sequence may be obtained as a restriction endonuclease fragment from any gene encoding a protein that has a signal sequence. Suitable prokaryotic signal sequences may be obtained from genes encoding, for example, LamB or OmpF (Wong et al., Gene, 68:1931 (1983), MalE, PhoA and other genes. Additional elements might include enhancers, Kozak sequences and intervening sequences flanked by donor and acceptor sites for RNA splicing. Highly efficient transcription can be achieved with the early and late promoters from SV40, the long terminal repeats (LTRs) from retroviruses, e.g., RSV, HTLVI, HIVI, and the early promoter of the cytomegalovirus (CMV). However, cellular elements can also be used (e.g., the human actin promoter). Suitable expression vectors for use in practicing the present invention include, for example, vectors such as pSVL and pMSG (Pharmacia, Uppsala, Sweden), pRSVcat (ATCC 37152), pSV2dhfr (ATCC 37146) and pBC12MI (ATCC 67109). The co-transfection with a selectable marker such as dhfr, gpt, neomycin, hygromycin genes for eukaryotic cells or tetracycline, kanamycin or ampicillin resistance genes for culturing in *E. coli* and other bacteria allows the identification and isolation of the transfected cells. The transfected nucleic acid can also be amplified to express large amounts of the encoded (poly)peptide. The DHFR (dihydrofolate reductase) marker is useful to develop cell lines that carry several hundred or even several thousand copies of the gene of interest. Another useful selection marker is the enzyme glutamine synthase (GS) (Murphy et al. 1991; Bebbington et al. 1992). Using these markers, the mammalian cells are grown in selective medium and the cells with the highest resistance are selected.

The coding sequences inserted in the vector can e.g. be synthesized by standard methods, or isolated from natural sources or produced semi-synthetically, i.e. by combining chemical synthesis and recombinant techniques. Ligation of the coding sequences to transcriptional regulatory elements and/or to other amino acid encoding sequences can be carried out using established methods.

The nucleic acid molecules of the invention as described herein above may be designed for direct introduction or for introduction via liposomes, phage vectors or viral vectors (e.g. adenoviral, retroviral) into the cell. Additionally, baculoviral systems or systems based on vaccinia virus or Semliki Forest virus can be used as eukaryotic expression system for the nucleic acid molecules of the invention. Expression vectors derived from viruses such as retroviruses, vaccinia virus, adeno-associated virus, herpes viruses, or bovine papilloma virus, may be used for delivery of the polynucleotides or vector into targeted cell population. Methods which are well known to those skilled in the art can be used to construct recombinant viral vectors; see, for example, the techniques described in Sambrook, 2001 and Ausubel, Current Protocols in Molecular Biology, Green Publishing Associates and Wiley Interscience, N.Y. (2001).

The present invention also relates to a non-human host transformed with the nucleic acid molecule or the vector of the invention.

Said host may be produced by introducing the nucleic acid molecule or the vector of the invention into a host, which upon its presence mediates the expression of the polypeptide encoded by the nucleic acid molecule or the vector.

In a preferred embodiment, the host is a cell, such as an isolated cell which may be part of a cell culture.

Suitable prokaryotic host cells comprise e.g. bacteria of the species Escherichia, *Bacillus*, *Streptomyces* and *Salmonella typhimurium.* Suitable eukaryotic host cells are e.g. fungal cells, inter alia, yeasts such as *Saccharomyces cerevisiae* or *Pichia pastoris* or insect cells such as Drosophila S2 and Spodoptera Sf9 cells and plant cells as well as mammalian cells. Mammalian host cells include without being limiting human HEK293, Hela, H9 and Jurkat cells, mouse NIH3T3 and C127 cells, COS 1, COS 7 and CV1, quail QC1-3 cells, mouse L cells, Chinese hamster ovary (CHO) cells and Bowes melanoma cells. The host cell may also be a primary cell or primary cell line. Primary cells are cells which are directly obtained from an organism. Suitable primary cells are, for example, mouse embryonic fibroblasts, mouse primary hepatocytes, cardiomyocytes and neuronal cells as well as mouse muscle stem cells (satellite cells) and stable, immortalized cell lines derived thereof.

It will readily be understood by the skilled person that the choice of host cell may be adjusted to achieved the desired posttranscriptional modification of the polypeptide of the invention, such as for example the presence or absence of glycosylation. For example, eukaryotic host cells mutated with respect to its ability to glycosylated may be employed, see e.g. Deutscher *et al*. 1984.

Appropriate culture media and conditions for the above-described host cells are known in the art and include, without being limiting, the conditions and media detailed further below.

The present invention further relates to a method for the production of a recombinant polypeptide having cytokine, preferably interleukin-10 function comprising culturing the host or the host cell of the invention under suitable conditions and isolating the polypeptide having cytokine, preferably interleukin-1 0 function produced.

Suitable conditions for culturing a prokaryotic or eukaryotic host are well known to the person skilled in the art. For example, suitable conditions for culturing bacteria are growing them under aeration in Luria Bertani (LB) medium. To increase the yield and the solubility of the expression product, the medium can be buffered or supplemented with suitable additives known to enhance or facilitate both. *E. coli* can be cultured from 4 to about 37°C, the exact temperature or sequence of temperatures depends on the molecule to be over-expressed. In general, the skilled person is also aware that these conditions may have to be adapted to the needs of the host and the requirements of the polypeptide expressed. In case an inducible promoter controls the nucleic acid molecule of the invention in the vector present in the host cell, expression of the polypeptide can be induced by addition of an appropriate inducing agent. Suitable expression protocols and strategies are known to the skilled person.

Depending on the cell type and its specific requirements, mammalian cell cultures can e.g. be carried out in RPMI or DMEM medium containing 10% (v/v) FCS, 2mM L-glutamine and 100 U/ml penicillin/streptomycine. The cells can be kept at 37 °C in a 5% CO₂, water saturated atmosphere. Suitable media for insect cell culture is e.g. TNM + 10% FCS or SF900 medium. Insect cells are usually grown at 27 °C as adhesion or suspension culture.

Further suitable expression protocols for eukaryotic cells are well known to the skilled person and can be retrieved e.g. from Sambrook, 2001, loc cit.

Methods of isolating the polypeptide produced are well-known in the art and comprise, without being limiting, method steps such as ion exchange chromatography, gel filtration chromatography (size exclusion chromatography), affinity chromatography, high pressure liquid chromatography (HPLC), reversed phase HPLC, disc gel electrophoresis or immunoprecipitation, see, for example, in Sambrook, 2001, loc. cit.

In addition to recombinant production, the polypeptide of the invention may be produced synthetically, e.g. by direct peptide synthesis using solid-phase techniques (cf Stewart et al. (1969) Solid Phase Peptide Synthesis; Freeman Co, San Francisco; Merrifield, J. Am. Chem. Soc. 85 (1963), 2149-2154). Synthetic protein synthesis may be performed using manual techniques or by automation. Automated synthesis may be achieved, for example, using the Applied Biosystems 431A Peptide Synthesizer (Perkin Elmer, Foster City CA) in accordance with the instructions provided by the manufacturer. Various fragments may be chemically synthesized separately and combined using chemical methods to produce the full length molecule. As indicated above, chemical synthesis, such as the solid phase procedure described by Houghton (Proc. Natl. Acad. Sci., 1985, 82: 5131) can be used.

A further, alternative, method for producing the polypeptide in accordance with the invention is *in vitro* translation of mRNA. Suitable cell-free expression systems for use in accordance with the present invention include rabbit reticulocyte lysate, wheat germ extract, canine pancreatic microsomal membranes, *E. coli* S30 extract, and coupled transcription/transiation systems such as the TNT-system (Promega). These systems allow the expression of recombinant polypeptides upon the addition of cloning vectors, DNA fragments, or RNA sequences containing coding regions and appropriate promoter elements.

The present invention further relates to a pharmaceutical composition comprising at least one of (a) the polypeptide of the invention; (b) the nucleic acid molecule of the invention; (c) the vector of the invention; and/or (d) the host of the invention.

In accordance with the present invention, the term "pharmaceutical composition" relates to a composition for administration to a patient, preferably a human patient. The pharmaceutical composition of the invention comprises the compounds recited above, alone or in combination. It may, optionally, comprise further molecules capable of altering the characteristics of the compounds of the invention thereby, for example, stabilizing, modulating and/or activating their function. The composition may e.g. be in solid or liquid form and may be, *inter alia*, in the form of (a) powder(s), (a) tablet(s), (a) solution(s) or (an) aerosol(s). The pharmaceutical composition of the present invention may, optionally and additionally, comprise a pharmaceutically acceptable carrier. By "pharmaceutically acceptable carrier" is meant a non-toxic solid, semisolid or liquid filler, diluent, encapsulating material or formulation auxiliary of any type. Examples of suitable pharmaceutically acceptable carriers are well known in the art and include phosphate buffered saline solutions, water, emulsions, such as oil/water emulsions, various types of wetting agents, sterile solutions, organic solvents including DMSO etc. Compositions comprising such carriers can be formulated by well known conventional methods.

These pharmaceutical compositions can be administered to the subject at a suitable dose. The dosage regimen will be determined by the attending physician and clinical factors. As is well known in the medical arts, dosages for any one patient depend upon many factors, including the patient's size, body surface area, age, the particular compound to be administered, sex, time and route of administration, general health, and other drugs being administered concurrently. The therapeutically effective amount for a given situation will readily be determined by routine experimentation and is within the skills and judgement of the ordinary clinician or physician. The skilled person knows that the effective amount of a pharmaceutical composition administered to an individual will, inter alia, depend on the nature of the compound. For example, if said compound is a polypeptide, the total pharmaceutically effective amount of pharmaceutical composition administered parenterally per dose will be in the range of about 1 µg protein/kg/day to 10 mg protein /kg/day of patient body weight, although, as noted above, this will be subject to therapeutic discretion. More preferably, this dose is at least 0.01 mg protein/kg/day, and most preferably for humans between about 0.01 and 1 mg protein/kg/day. The length of treatment needed to observe changes and the interval following treatment for responses to occur vary depending on the desired effect. The particular amounts may be determined by conventional tests which are well known to the person skilled in the art.

Pharmaceutical compositions of the invention may for example be administered orally, rectally, parenterally, intracisternally, intraperitoneally, topically (as by powders, ointments, drops or transdermal patch), bucally, or as a nasal spray. The term "parenteral" as used herein refers to modes of administration, which include intravenous, intramuscular, intrasternal, subcutaneous and intraarticular injection and infusion.

It will be understood by the skilled person that when the pharmaceutical composition comprises the nucleic acid molecule of the invention, said nucleic acid molecule is provided in a suitable form to ensure expression of the polypeptide of the invention. For example, the nucleic acid molecule may be operatively linked to a promoter or may be provided in form of the vector or the host of the invention.

The present invention further relates to the polypeptide, the nucleic acid molecule, the vector or the host of the present invention for use in treating and/or preventing inflammatory diseases.

"Inflammatory diseases" include all diseases associated with acute or chronic inflammation. Acute inflammation is the initial response of the body to harmful stimuli and results from an increased movement of plasma and leukocytes (such as e.g. granulocytes) from the blood into the injured tissues. A number of biochemical events propagates and matures the inflammatory response, involving the local vascular system, the immune system, and various cells within the injured tissue. Prolonged inflammation is referred to as chronic inflammation, which leads to a progressive shift in the type of cells present at the site of inflammation and is characterized by simultaneous destruction and healing of the tissue from the inflammatory process. Inflammatory diseases can be caused by e.g. burns, chemical irritants, frostbite, toxins, infection by pathogens, physical injury, immune reactions due to hypersensitivity, ionizing radiation, or foreign bodies, such as e.g. splinters, dirt and debris. Examples of inflammatory diseases are well known in the art.

It is understood also in accordance with this embodiment of the invention that expression of the polypeptide is required from the nucleic acid molecule, the vector or the host of the present invention in order to obtain the claimed use. Suitable means to ensure expression of the polypeptide from the nucleic acid molecule, the vector or the host of the present invention are known to the skilled person.

In a preferred embodiment, the inflammatory disease is selected from the group consisting of inflammatory bowel disease, rheumatoid arthritis, psoriasis and bacterial sepsis.

The term "inflammatory bowel disease", as used herein, refers to a group of inflammatory conditions of the colon and small intestine including for example Crohn's disease, ulcerative colitis, collagenous colitis, lymphocytic colitis, ischaemic colitis, diversion colitis, Behçet's syndrome and indeterminate colitis.

"Rheumatoid arthritis", in accordance with the present invention, is an autoimmune disorder that causes the body's immune system to attack the bone joints (Muller B et al. 1998. Springer Semin Immunopathol. 20:181-96). Rheumatoid arthritis is a chronic, systemic inflammatory disorder that may affect many tissues and organs, but principally attacks synovial joints. The process produces an inflammatory response of the synovium (synovitis) secondary to hyperplasia of synovial cells, excess synovial fluid, and the development of pannus in the synovium. The pathology of the disease process often leads to the destruction of articular cartilage and ankylosis of the joints. Rheumatoid arthritis can also produce diffuse inflammation in the lungs, pericardium, pleura, and sclera, and also nodular lesions, most common in subcutaneous tissue under the skin.

"Psoriasis", in accordance with the present invention, is a disease which affects the skin and joints. It commonly causes red scaly patches to appear on the skin. The scaly patches caused by psoriasis, called psoriatic plaques, are areas of inflammation and excessive skin production. Skin rapidly accumulates at these sites and takes a silvery-white appearance. Plaques frequently occur on the skin of the elbows and knees, but can affect any area including the scalp and genitals. Psoriasis is hypothesized to be immune-mediated and is not contagious. The disorder is a chronic recurring condition which varies in severity from minor localised patches to complete body coverage. Fingernails and toenails are frequently affected (psoriatic nail dystrophy) - and can be seen as an isolated finding. Psoriasis can also cause inflammation of the joints, which is known as psoriatic arthritis. Ten to fifteen percent of people with psoriasis have psoriatic arthritis.

The term "bacterial sepsis", as used herein, refers to life-threatening conditions resulting from the circulation of bacteria in the blood stream. Sepsis results in generalized systemic production of proinflammatory cytokines that results in tissue damage and ultimately septic shock due to failure of the microcirculation.

The present invention further relates to an inhibitor of cytokine receptor activity, wherein the inhibitor is a polypeptide comprising two cytokine monomer subunits covalently linked by a linker, wherein (a) one monomer binds to a receptor chain of the cytokine receptor to be inhibited and the second monomer does not bind to a receptor chain of said cytokine receptor; and/or (b) the linker interferes with dimerisation of the cytokine receptor chains of the receptor to be inhibited.

The term "inhibitor", in accordance with the present invention, relates to a polypeptide having the above defined structure and reducing the biological activity of a cytokine receptor by at least 50%, preferably by at least 75%, more preferred by at least 90% and even more preferred by at least 95% such as at least 98% or even at least 99%, such as 100%. A reduction by e.g. 98% denotes that only 2% of the biological activity of the receptor are maintained in the presence of the inhibitor as compared to the biological activity of said receptor in the absence of the inhibitor. Biological function denotes in particular any known biological function of cytokine receptors. Examples of said biological function are provided herein above. All these functions can be tested for by the skilled person either on the basis of common general knowledge or on the basis of the teachings of this specification, optionally in conjunction with the teachings of the documents cited therein.

Unless explicitly stated otherwise, the definitions as well as the preferred embodiments provided herein above with regard to all other embodiments such as e.g. polypeptide of the invention, the nucleic acid molecule of the invention, the host of the invention etc. apply mutatis mutandis also to the embodiments relating to the inhibitor of cytokine receptor activity as outlined above. For example, preferred embodiments of the polypeptide have their counterparts in preferred embodiments of the above defined inhibitor.

In accordance with option (a) of this embodiment of the invention, it is envisaged that either (i) the second monomer is not able to bind to a receptor chain of said cytokine receptor at all or (ii) that the second monomer binds to a receptor chain of a different cytokine receptor.

The terms "receptor chain of said cytokine receptor" as well as "the same cytokine receptor chain", as used herein, relate to a chain of the same cytokine receptor type, such as for example an IL-10 receptor chain and of the same species. Many cytokines activate their receptor by first binding to a first ligand binding receptor chain, generating a complex of a cytokine monomer and a first receptor chain. In a second step, another receptor chain is occupied by a second cytokine monomer. Then both occupied receptor chains come together and in the cytokine binding area both cytokine molecules form a dimer. Cross-linking occurs and the cytokine receptor signals, usually via another cytokine receptor chain that binds to the cytokine ligand binding chain complex. Thus, two separate chains of the same type of cytokine receptor have to be bound by cytokine monomers in order to achieve activation of the receptor. Accordingly, the term "a different cytokine receptor", as used herein, encompasses cytokine receptor chains that are receptor chains for different cytokine, such as IL-10 and IL-6, but also cytokine receptor chains for the same cytokine, such as e.g. IL-10, but of a different species.

A "monomer that is not able to bind to a receptor chain of said cytokine receptor" in accordance with option (i) above may e.g. be a human IL-10 monomer comprising mutations that result in the monomer not being able to bind to the IL-10 receptor chain any longer. In accordance with option (ii), a dimer may be employed having e.g. an IL-10 monomer covalently linked to an IL-6 monomer. Both monomer subunits will bind to their respective cytokine receptor chains, thus resulting in an inhibition of both signalling pathways. As a further option, a covalently linked cytokine dimer comprising e.g. a human IL-10 monomer and a mouse IL-10 monomer may be employed (fulfilling the requirements of both option (i) and option (ii)). Such a dimer will result in the inhibition of human IL-10 receptor signalling, as the mouse IL-10 monomer is not able to bind to the IL-10 receptor.

In accordance with option (b) of this embodiment of the invention, the linker that covalently links the two cytokine monomers interferes with dimerisation of the cytokine receptor chains of the receptor to be inhibited. As defined herein above, the linker may be a peptide or a non-peptide linker in accordance with the above detailed definitions. However, the considerations for linker design in accordance with option (b) of this embodiment of the inhibitor of the invention are directed to a sterical hindrance of receptor dimerisation. Thus, the linker may e.g. be designed to have certain hydrophobic or charged moieties that result in interaction with the functional protein domains of the receptor chains, while not interfering with dimerisation of the inhibitor of the invention.

As outlined above, the skilled person knows how to design appropriate linker molecules based on his/her common knowledge. For example, peptide linkers can be chosen from the LIP (Loops in Proteins) database (Michalsky et al., 2003) obtained commercially (see, for example, the catalogue from Glen Research, 22825 Davis Drive, Sterling, Virginia, 20164 USA). Furthermore, the above outlined methods for testing the suitability of different linkers apply also to this embodiment, e.g. the properties of the linker may be tested by assessing the activation (or lack thereof) of the target receptor in the presence of the inhibitor of the present invention as compared to activation of the target receptor in the presence of a known ligand, such as for example a wild-type cytokine known to induce receptor activity. The stability of the resulting molecule can also be measured, as outlined above, by e.g. melting point analysis in circular dichroism spectroscopy.

In a preferred embodiment of the inhibitor of the invention, the polypeptide comprises a human IL-10 monomer and a mouse IL-10 monomer covalently linked by a linker.

Unless otherwise defined, all technical and scientific terms used herein have the same meaning as commonly understood by one of ordinary skill in the art to which this invention belongs. In case of conflict, the patent specification, including definitions, will prevail.

The figures show:
**Figure 1****: Stabilized IL-10 homodimer (the IL-10 of the invention).** (A) The ribbon diagram shows the structure of the IL-10 dimer bound to two chains of IL-10R1 (taken from Josephson et al., 2001, Immunity 14: 35-46). The C-terminus of one IL-10 monomer is located in close proximity to the N-terminus of the second monomer. The arrow indicates the position of the peptide linker, which connects the monomers C-terminal to N-terminal. (B) Design of recombinant stabilized IL-10 dimer expressed as a continuous polypeptide. The arrows each represent a wildtype murine IL-10 monomer. The 7 amino acid linker and its sequence are indicated in light grey.
**Figure 2****. Expression of the stabilized recombinant IL-10 dimer in mammalian cells in vitro.** A) The eukaryotic expression vector with the inserted cDNA encoding the IL-10 fusion protein ("stable IL-10"). The protein expression plasmid pcepPu contains a thrombin-cleavable Histidin (His)-tag. oriP, origin of replication, pUC ori, ColE1 origin of replication, pCMV, cytomegalovirus promotor, SV40pA, simian-virus 40 polyadenylation signal, BM40, signal peptide of the BM40 protein, EBNA-1, Epstein-Barr-virus nuclear antigen 1. The vector was transfected into human embryonic kidney (HEK)-293 cells. The identical vector containing a single wildtype IL-10 cDNA was transfected in parallel. B) Western blot of wt and "stable IL-10" purified from supernatant of the HEK-293 cells.
**Figure 3****: Establishment of an TNFa suppression bioassay for IL-10 activity.** Short-term cultures of mouse splenocytes were incubated with LPS and either recombinant wildtype mouse IL-10 expressed in human HEK293 cells or commercial bacterially expressed mouse IL-10 for 24 hours. Concentrations of TNFα in the supernatant was quantified by ELISA. Note that the wt IL-10 is active, while the commercial IL-10 protein shows only marginal suppression of TNF release, which may be potentially due to incomplete dimerization of the commercial protein.
**Figure 4****: Comparison of wildtype IL-10 and the IL-10 of the invention for bioactivity in the TNFa-suppression assay.** Fresh mouse splenocyte suspensions were generated and equal numbers of total spleen cells incubated for 24 h at 37°C with LPS and different concentrations of the IL-10 of the invention or wildtype IL-10. Supernatant was harvested and TNFα concentrations were determined by ELISA. Note that "stable" IL-10 of the invention is significantly more effective in TNFα suppression.

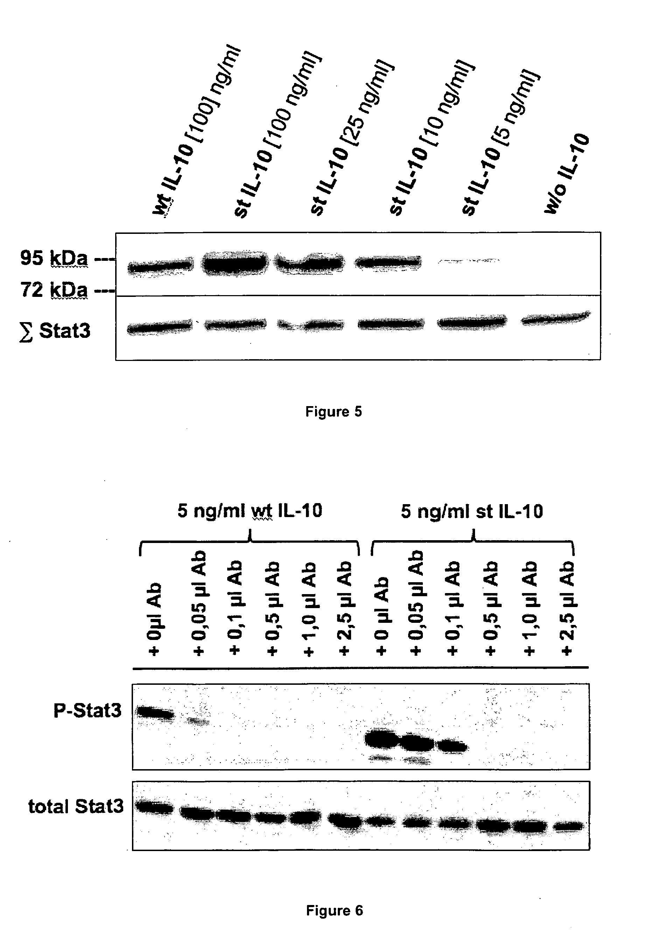

**Figure 5****. Bioassay for IL-10 bioactivity: Induction of Stat3-Phosphorylation.** Fresh mouse splenocyte suspensions were generated and equal numbers of total spleen cells incubated for 30 min at 37°C with different concentrations of "stable" or wildtype IL-10. Cells were lysed by RIPA-buffer containing phosphatase and protease inhibitors. Lysates were loaded onto a polyacrylamide gel under reducing conditions. The gel was blotted onto a PVDF membran and probed with an anti phospho-Stat3 antibody and a swine anti-rabbit HRP-coupled secondary antibody. The expected size for Stat3 is 86kDa. For loading control, the membrane was stripped and re-probed with mouse anti-Stat3 and goat-anti-mouse Ig coupled to HRP to detect total Stat3. Note the dose dependent induction of Stat3 phosphorylation by the IL-10 of the invention. 10 ng/ml induce a similar amount of phospho-Stat3 as 100 ng/ml wt IL-10.
**Figure 6****. Induction of P-Stat3 by recombinant wt or "stable" IL-10 can be blocked by anti-IL-10 antibody.** Assay as in figure 5. The recombinant IL-10 was pre-incubated with anti-IL-10 before addition to the cells. Note that 5 ng/ml the "stable" (st) IL-10 of the invention results in more pronounced Stat3 phosphorylation as compared to the same amount of wt IL-10. Higher amounts of anti-IL-10 are required to block induction of Stat3 phosphorylation by the IL-10 of the invention as compared to wt IL-10.

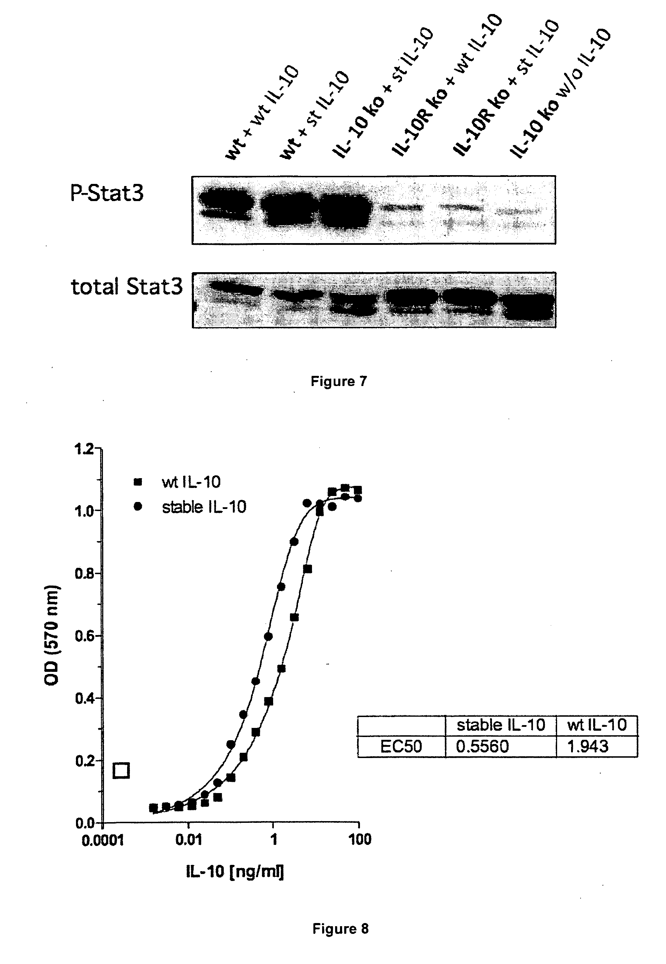

**Figure 7****: Induction of P-Stat3 by recombinant "stable" or wt IL-10 does not occur in IL-10R-deficient cells.** Experimental setup as in Figures 5 and 6
**Figure 8****: Comparison of wildtype IL-10 and the IL-10 of the invention for the capacity to induce proliferation of Ba/F3 cells in vitro.** Ba/F3 cells were incubated with two-fold serial dilutions of wt and "stable" IL-10 for 48 hours at 37°C, 5% CO₂. Viable cells were stained by the dye MTT (3-(4,5-Dimethylthiazol-2-yl)-2,5-diphenyltetrazoliumbromid) and quantified by absorbance measurement (570 nm)

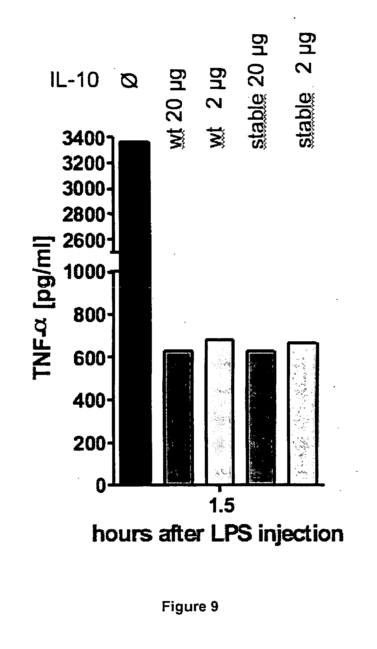

**Figure 9****: Suppression of the systemic inflammatory response to LPS by wildtype IL-10 and the IL-10 of the invention.** BALB/c wildtype mice were intravenously injected with recombinant IL-10 followed by injections of LPS 30 min later. After 1.5 hours, serum concentrations of TNFα were assayed by ELISA. At the concentrations tested, which seem to be saturating, both wt and "stable" IL-10 show similar potential to suppress the inflammatory response.

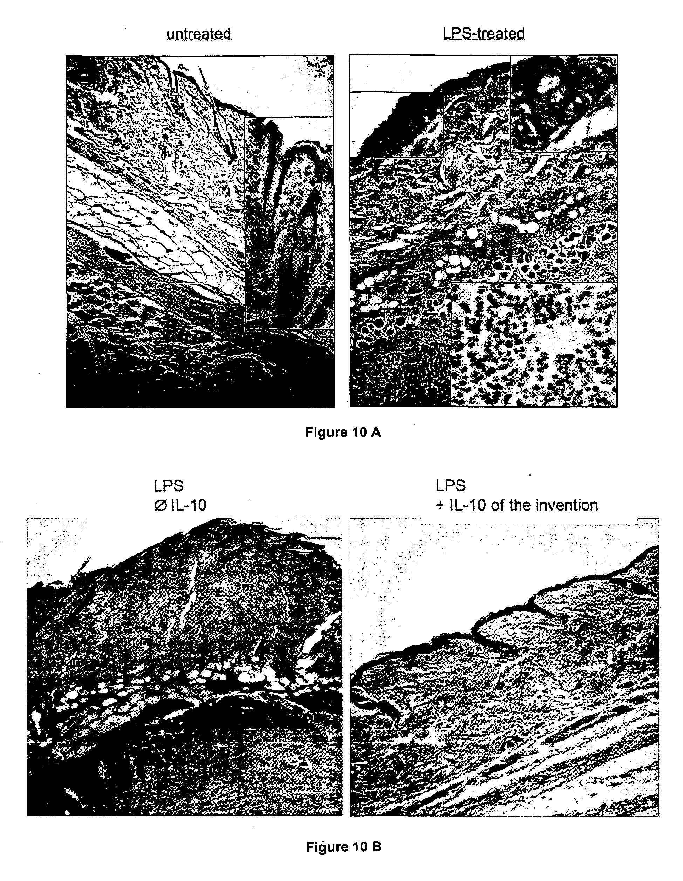

Figure 10: **Suppression of the local LPS-induced tissue inflammation by wildtype IL-10 and the IL-10 of the invention.** LPS (10 µg) was injected subcutaneously once per day for two consecutive days at the same site on the flank of IL-10-/- mice. On day four after the first injection, the skin was excised formalin-fixed paraffin-embedded. Sections were stained with Hematoxilin & Eosin. A) Massive inflammatory infiltrate and extensive necrosis of epidermal and dermal structures and the panniculus carnosus (right panel). Normal mouse skin is shown for comparison (left panel). At the margins of the inflammatory lesion, the epithelium displays a reactive hyplasia (upper left inset). Within the lesion, all epithelial structures are necrotic. The upper right inset shows remnants of hair follicles and the necrotic epidermis. The lower right inset shows the massive infiltration of the tissue with neutrophilic granulocytes. B) 2 µg recombinant wildtype IL-10 or IL-10 of the invention injected along with the LPS nearly completely blocked issue inflammation.

The examples illustrate the invention:

### Example 1: Material and Methods

### Cell line and Cytokine

Ba/F3 cells were provided by Kastelein R.A. (SCHERING-PLOUGH BIOPHARMA, Palo Alto) and maintained in Ba/F3 medium: RPMI 1640 supplemented with 10% FCS, 1% Penicillin Streptomycin (Biochrom), 2mM L-glutamin, 50µM b-mercaptoethanol and 10 ng/ml mouse IL-3 (Natutec). For the proliferation assay with IL-10 preparations, cells were collected by centrifugation, washed three times in Ba/F3 medium without IL-3, counted, and resuspended to the appropriate density for plating.

### Antibodies

The neutralizing antibody clone Jes5-2a5 (ebiosciences) was used to control for the specificity of effects induced by IL-10 preparations. For western blots, rabbit anti-phospho-Stat3 (cell signaling), mouse anti-Stat3 (Santa Cruz), swine anti-rabbit Hrp (DAKO) and goat anti mouse Hrp (R&D Systems) were used.

### Western blot

Tyrosine-phosphorylation of STAT-3 in mouse splenocytes was analysed by Western blot. C57/BL6 spleens were homogenized and red blood cells were lysed by short incubation in ammonium chloride buffer. Equal amounts of cells where mixed with different concentrations of the IL-10 of the invention and wildtype IL-10, incubated for 30 min at 37 °C and lysed by RIPA-buffer supplemented with phostop (Roche) and protease inhibitors (Complete, Roche). Cleared lysates were loaded onto a polyacrylamide gel under reducing conditions. The gel was blotted to a PVDF membrane and probed with anti phospho-Stat3 antibody and swine anti-rabbit HRP secondary antibody. For loading control, total STAT3 was detected after stripping by re-probing with mouse anti- STAT3 and goat anti-mouse HRP. Chemiluminescence (ECL, Pierce) was detected by X-ray films.

### Proliferation assay

The Ba/F3 proliferation assay was performed in 96-well flat bottom plates in Ba/F3 medium (without IL-3). The assay volume was 100 µL per well. Bioassay plates were incubated in a humidified atmosphere (37°C, 5% CO2) for 40-48 hours. IL-10 was prepared to working concentration and added first to the well. Serial dilutions of 1:2 were performed with Ba/F3 medium across the wells. Cells were suspended to the appropriate density for plating and added to the well. The proliferation rate was measured by adding MTT (Sigma) to a final concentration of 0.5 mg/ml. After 4 hours, cells were lysed by adding 100 µl of lysis buffer (0,1 N HCl, 10% SDS) and incubation over night. Absorbance was read at 570 nm.

### Suppression of TNF-release

### in vitro

Fresh mouse splenocytes were generated by pushing the spleen through a cell strainer. Cells were resuspended in IMDM medium (10% FCS, 1% Penicillin Streptomycin, 2 mM L-glutamin). Equal numbers of total spleen cells were plated in a 96-well plate and incubated with LPS (lipopolysaccharide) and different concentrations of "stable", wt or commercial IL-10 for 24 h at 37°C. Supernatant was harvested and TNFα concentrations were determined by ELISA.

### invivo

BALB/c wildtype mice of the same age and gender got inhalation anaesthesia and were intravenously injected with recombinant "stable" or wt IL-10 followed by injections of LPS 30 min later. After 1.5 h blood samples were taken from the retroorbital plexus. After complete coagulation serum samples were assayed by ELISA. Mice were always narcotized by inhalation anaesthesia before injections and blood taking

### Suppression of local skin inflammation

LPS (10 µg) was injected under skin of IL-10^{-/-} once per day for two consecutive days. In some cases LPS was mixed with high amounts of wt IL-10 and injected likewise. On day four after the first injection the skin was excised, fixed in formalin and processed for histology. Skin sections were stained with haematoxylin and eosin and were put onto an object slide for microscopy

### Example 2: Cloning

Total peritoneal lavage cells (macrophages, B cells and mast cells) from wildtype C57BL/6 mice were incubated at 37°C in RPMI 1640 medium. The cells were stimulated with 1 µg/ml LPS for 1 hour. Total RNA was isolated from the cells and reverse transcribed using oligo-dt primers. The IL-10 cDNA was amplified with specific primers containing a Nhel restriction site to the 5'and a BamHI restriction site to the 3'end. This PCR-product and the mammalian expression vector (see fig. 2) were both digested with Nhel and BamHI and purified by gel extraction. Then, T4-ligation of the IL-10 cDNA into the vector yielded the construct for expression of wildtype mouse IL-10, which was transformed into E. coli and isolated by alkaline lysis. In order to generate the construct encoding the IL-10 of the invention, a second IL-10 cDNA was ligated into the first construct and positioned behind the first cDNA. In a first step, IL-10 cDNA was amplified again, this time using a primer containing a BamHI site and the polypeptide linker and a primer containing a BamHI site. After BamHI restriction digest of this PCR product, it was ligated behind the IL-cDNA of the wildtype construct that was also cleaved by BamHI. The resulting construct (shown in figure 2) was also propagated in E.coli.

### Example 3: Expression and purification

Human embryonic kidney cells (Hek293) were transfected with the constructs encoding wildtype IL-10 or the IL-10 of the invention. Cells were propagated in DMEM/F12 Glutamax media (Invitrogen) supplemented with 10% FCS and 1% Penicillin Streptomycin (Biochrom). To select for the presence of the transfected plasmids, the antibiotic puromycin was added. After the propagation period, the cells were maintained in serum-free media. Every second day medium was harvested and stored at -20°C. Fresh medium was added. For protein purification, the frozen medium was thawed and loaded onto a nickel NTA-sepharose column (Amersham) exploiting the N-terminal histidin-tag of the recombinant proteins. After washing, the column was eluted with PBS buffer containing imidazol. The eluted fractions were analyzed on a polyacrylamid-gel. The IL-10-containing fractions were pooled and dialyzed against PBS buffer. Finally, the protein solution was sterile-filtered and stored in working aliquots at -80°C.

### Example 4: Bioactivity of the recombinant IL-10 proteins in vitro

In order to determine whether the recombinant IL-10 proteins were bioactive, three different *in vitro* assays for IL-10 activity were established. First, the suppression of TNFα production of splenocytes in response to LPS *in vitro* by recombinant IL-10 was determined. TNFα concentration in the supernatant of the short-term cultures was measured by ELISA. Interestingly, two samples of bacterially expressed commercial IL-10 (from two different manufacturers) did not show any or only marginal activity in this assay, while the recombinant wildtype IL-10 that we had expressed in human HEK293 cells clearly showed a dose-dependent activity (Figure 3). The comparison of recombinant wildtype IL-10 and the IL-10 of the invention demonstrated that the IL-10 of the invention showed robust activity that was slightly higher than that of wildtype IL-10 (Figure 4). The next assay exploits the fact that IL-10 signals via STAT3. STAT3 phosphorylation can be determined by Western blot analysis using a phospho-STAT3-specific antibody. As shown in Figure 5, phospho-STAT3 was undetectable in short-term *ex vivo* cultures of mouse splenocytes without addition of recombinant IL-10. Both, wildtype IL-10 and the IL-10 of the invention induced robust STAT3 phosphorylation. Interestingly, 10 ng/ml of the IL-10 of the invention induced a signal of the same intensity as 100 ng/ml of wildtype IL-10 indicating a roughly 10-fold higher activity of the IL-10 of the invention. The STAT3 phosphorylation observed indeed reflected specific IL-10 effects since it could be blocked completely by an anti-IL-10 antibody (Figure 6). Of note, significantly higher amounts of the antibody were required to block the signaling induced by the IL-10 of the invention as compared to wildtype IL-10. Furthermore, induction of STAT3 phosphorylation by recombinant IL-10 was not observed in IL-10R-/- cells providing further evidence for the specificity of the observed effect (Figure 7). Signaling was induced in IL-10-/- cells demonstrating that the signaling was indeed induced by the recombinant IL-10 and not by IL-10 produced by the cells. In addition, bioactivity of recombinant IL-10 proteins was quantified by the induction of proliferation of the IL-10-dependent cell line Ba/F3. As shown in figure 8, equal amounts of the IL-10 of the invention induced more vigorous Ba/F3 proliferation compared to wildtype IL-10.

### Example 5: Bioactivity of the IL-10 of the invention in vivo

In order to investigate whether the recombinant IL-10 proteins were active *in vivo,* two different assays were used. First, the IL-10 preparations were tested for their capacity to suppress the systemic inflammatory response to LPS. To this end, BALB/c mice were intravenously injected with recombinant IL-10 followed by i.v. injections of LPS 30 min later. After 1.5 hours, serum concentrations of TNFα were assayed by ELISA. At the concentrations tested, which seem to be saturating, both wt and the "stable" IL-10 of the invention show a comparable suppression of the inflammatory response (figure 9).

In order to also test the capacity of the recombinant IL-10 proteins to suppress local tissue inflammation, LPS was injected subcutaneously into IL-10-/- mice at the same site (flank) on day 1 and 2. On day 4, the resulting tissue inflammation was investigated by histological analysis of Hematoxilin-Eosin-stained paraffin sections. As shown in figure 10 A (right panel), the LPS injections resulted in a massive local inflammation with dense infiltrates of inflammatory cells (mostly neutrophilic granulocytes) that resulted in extensive necrosis of epidermis, adnexal structures and the panniculus carnosus. Co-injection of 2 µg of recombinant IL-10 (wildtype or IL-10 of the invention) along with the LPS suppressed this inflammatory response almost completely (n=3, figure 10 B). At a dose of 20 ng recombinant IL-10, the suppression was clearly more pronounced for the IL-10 of the invention as compared to wildtype IL-10. However, this result is preliminary, since this dose was tested on one mouse for each of the two IL-10 preparations only.

Collectively, these data show that the IL-10 of the invention is active *in vivo* after systemic or local administration. Future experiments will address whether the IL-10 of the invention is even more active than wildtype IL-10.

### References

Anderson CF, Oukka M, Kuchroo VJ, Sacks D. CD4(+)CD25(-)Foxp3(-) Th1 cells are the source of IL-10-mediated immune suppression in chronic cutaneous leishmaniasis. J Exp Med 2007; 204: 285-97.
Asadullah K, Sterry W, Volk HD. Interleukin-10 therapy--review of a new approach. Pharmacol Rev. 2003;55:241-69.
Berg DJ, Kühn R, Rajewsky K, Müller W, Menon S, Davidson N, Grünig G, Rennick D. Interleukin-10 is a central regulator of the response to LPS in murine models of endotoxic shock and the Shwartzman reaction but not endotoxin tolerance. J Clin Invest. 199;96:2339-47.
Blancho et al., 1995 Proc. Natl. Acad. Sci U.S.A. 92:2800
Deutscher et al. 1984, "Translocation at golgi vesicle membranes: a CHO glycosylation mutant deficient in CMP-sialic acid transport, Cell 39(2):295-299.
de Waal Malefyt R, Abrams J, Bennett B, Figdor CG, de Vries JE. Interleukin 10(IL-10) inhibits cytokine synthesis by human monocytes: an autoregulatory role of IL-10 produced by monocytes. J Exp Med 1991; 174: 1209-20.
Eming SA, Werner S, Bugnon P, Wickenhauser C, Siewe L, Utermöhlen O, Davidson JM, Krieg T, Roers A. Accelerated wound closure in mice deficient for interleukin-10. Am J Pathol. 2007;170:188-202.
Fillatreau S, Sweenie CH, McGeachy MJ, Gray D, Anderton SM. B cells regulate autoimmunity by provision of IL-10. Nat Immunol 2002; 3: 944-50.
Fiorentino DF, Zlotnik A, Mosmann TR, Howard M, O'Garra A. IL-10 inhibits cytokine production by activated macrophages. J Immunol 1991; 147: 3815-22.
Fleming SB, McCaughan CA, Andrews AE, Nash AD, and Mercer AA (1997) A homolog of interleukin-10 is encoded by the poxvirus orf virus. J Virol 71:4857 - 4861.
Glocker EO, et al., Inflammatory bowel disease and mutations affecting the interleukin-10 receptor. N Engl J Med. 2009;361:2033-45.
Hsu DH, De Waal Malefyt R, Fiorentino DF, Dang MN, Vieira P, De Vries J, Spits H, Mosmann TR, and Moore KW (1990) Expression of interleukin-10 activity by Epstein-Barr virus protein BCRF1. Science (Wash DC) 250:830-832.
Kotenko SV, Saccani S, Izotova LS, Mirochnitchenko OV, and Pestka S (2000) Human cytomegalovirus harbors its own unique IL-10 homolog (cmv IL-10). Proc Natl Acad Sci USA 97:1695-1700.
Lutolf MP, Hubbell JA. Synthetic biomaterials as instructive extracellular microenvironments for morphogenesis in tissue engineering. Nat Biotechnol. 2005;23:47-55.
Igyarto BZ, Jenison MC, Dudda JC, Roers A, Müller W, Koni PA, Campbell DJ, Shlomchik MJ, Kaplan DH. Langerhans cells suppress contact hypersensitivity responses via cognate CD4 interaction and langerhans cell-derived IL-10. J Immunol. 2009;183:5085-93.
Jankovic D, Kuliberg, MC, Feng CG et al. Conventional T-bet+Foxp3- Th1 cells are the major source of host-protective regulatory IL-10 during intracellular protozoan infection. J Exp Med 2007; 204: 273-83.
Kang K, Hammerberg C, Meunier L, Cooper KD. CD11b+ macrophages that infiltrate human epidermis after in vivo ultraviolet exposure potently produce IL-10 and represent the major secretory source of epidermal IL-10 protein. J Immunol. 1994;153:5256-64.
Kühn R, Löhler J, Rennick D, Rajewsky K, Müller W. Interleukin-10-deficient mice develop chronic enterocolitis. Cell. 1993;75:263-74.
Langley et al. 2001 gene bank accession AAK94013
Lu et al., 1995 J. Interf. Cytokine Res. 15:1103
Madan R, et al., Nonredundant roles for B cell-derived IL-10 in immune counter-regulation. J Immunol. 2009;183:2312-20.
Mangan NE, Fallon RE, Smith P, van Rooijen N, McKenzie AN, Fallon PG. Helminth infection protects mice from anaphylaxis via IL-10-producing B cells. J Immunol 2004; 173: 6346-56.
Mauri C, Gray D, Mushtaq N, Londei M. Prevention of arthritis by interleukin 10-producing B cells. J Exp Med 2003; 197: 489-501.
Mizoguchi A, Mizoguchi E, Takedatsu H, Blumberg RS, Bhan AK. Chronic intestinal inflammatory condition generates IL-10-producing regulatory B cell subset characterized by CD1d upregulation. Immunity 2002; 16: 219-30.
Moore K, Vieira P, Fiorentino DF, Trounsine ML, Khan TA, Mosmann TR. Homology of Cytokine Synthesis Inhibitory Factor (IL-10) to the Epstein-Barr Virus Gene BCRFI. Science, 1990; 248(4960): 1230-1234
Moore KW, de Waal Malefyt R, Coffman RL, O'Garra A Interleukin-10 and the interleukin-10 receptor. Annu Rev Immunol. 2001;19:683-765.
O'Garra A, Chang R, Go N, Hastings R, Haughton G, Howard M. Ly-1 B (B-1) cells are the main source of B cell-derived interleukin 10. Eur J Immunol 1992; 22: 711-17.
Rode HJ, Bugert JJ, Handermann M, Schnitzler P, Kehm R, Janssen W, Delius H, and Darai G (1994) Molecular characterization and determination of the coding capacity of the genome of equine herpesvirus type 2 between the genome coordinates 0.235 and 0.258 (the EcoRI DNA fragment N; 4.2kbp). Virus Genes 9:61-75.
Roers A, Siewe L, Strittmatter E et al. T cell-specific Inactivation of the interleukin-10 gene in mice results in enhanced T cell responses but normal innate responses to lipopolysaccharide or skin irritation. J Exp Med 2004; 200: 1289-97.
Rubtsov YP, Rasmussen JP, Chi EY, Fontenot J, Castelli L, Ye X, Treuting P, Siewe L, Roers A, Henderson WR Jr, Muller W, Rudensky AY Immunity. Regulatory T cell-derived interleukin-10 limits inflammation at environmental interfaces. 2008;28:546-58.
Scarozza et al., 1998 Cytokine 10:851
Siewe L, Bollati-Fogolin M, Wickenhauser C, Krieg T, Müller W, Roers A. Interleukin-10 derived from macrophages and/or neutrophils regulates the inflammatory response to LPS but not the response to CpG DNA. Eur J Immunol. 2006;36:3248-55.
Spencer JV, Lockridge KM, Barry PA, Lin G, Tsang M, Penfold ME, and Schall TJ (2002) Potent immunosuppressive activities of cytomegalovirus-encoded IL-10. J Virol 76:1285-1292.
Sutterwala FS, Noel GJ, Salgame P, Mosser DM.J Exp Med. Reversal of proinflammatory responses by ligating the macrophage Fcgamma receptor type I. 1998;188:217-22.
Vieira P, de Waal-Malefyt R, Dang MN, Johnson KE, Kastelein R, Fiorentino DF, deVries JE, Roncarolo MG, Mosmann TR, Moore KW (1991). Isolation and expression of human cytokine synthesis inhibitory factor cDNA clones: homology to Epstein-Barr virus open reading frame BCRFI. PNAS 88(4): 1172-1176.
Villinger et al., 1995 J. Immunol. 155: 3946

## Claims

1. Polypeptide having interleukin-10 function, comprising two interleukin-10 monomer subunits covalently linked by a linker.

2. The polypeptide of claim 1, wherein the interleukin-10 monomers are independently selected from the group consisting of mammalian- and virus-derived interleukin-10 monomers.

3. The polypeptide of claim 1 or 2, wherein the interleukin-10 monomers are independently selected from (i) anyone of SEQ ID NOs:1 to 7, and/or (ii) a sequence having at least 70% sequence identity to SEQ ID NO:1 and essentially retaining the biological activity of IL-10.

4. The polypeptide according to any one of claims 1 to 3, wherein the linker is a peptide linker.

5. The polypeptide according to claim 4, which is a single polypeptide chain.

6. The polypeptide according to claim 4 or 5, wherein the peptide linker is selected from the group consisting of (Gly₃SerGly₃) ₙ, (Gly₂Ser₁Gly₂) ₙ, (GlySer₁Gly)ₙ, and (Gly₃Ser₁Gly₂)ₙ, wherein n is an integer independently selected from 0, 1, 2, 3 or 4.

7. The polypeptide according to any one of claims 1 to 6, wherein the linker is covalently linked to the C-terminus of one interleukin-10 monomer subunit and the N-terminus of the second interleukin-10 monomer subunit.

8. A nucleic acid molecule encoding the polypeptide of claim 5 or 6.

9. A vector comprising the nucleic acid molecule of claim 8.

10. A non-human host transformed with the nucleic acid molecule of claim 8 or the vector of claim 9.

11. The non-human host of claim 10, wherein the host is a cell.

12. A method for the production of a recombinant polypeptide having interleukin-10 function comprising culturing the host cell of claim 11 under suitable conditions and isolating the polypeptide having interleukin-10 function produced.

13. A pharmaceutical composition comprising at least one of
(a) the polypeptide of any one of claims 1 to 7;
(b) the nucleic acid molecule of claim 8;
(c) the vector of claim 9; and/or
(d) the host of claim 10 or 11.

14. The polypeptide according to any one of claims 1 to 7, the nucleic acid molecule of claim 8, the vector of claim 9 or the host of claim 10 or 11 for use in treating and/or preventing inflammatory diseases.

15. The polypeptide, the nucleic acid molecule, the vector or the host of claim 14, wherein the inflammatory disease is selected from the group consisting of inflammatory bowel disease, rheumatoid arthritis, psoriasis and bacterial sepsis.


SEQUENCE LISTING

<110> Technische Universität Dresden, University of Manchester

<120> Covalently linked interleukin-10

<130> S2944 EP

<160> 16

<170> PatentIn version 3.4

<210> 1
<211> 160
<212> PRT
<213> homo sapiens

<400> 1

Ser Pro Gly Gln Gly Thr Gln Ser Glu Asn Ser Cys Thr His Phe Pro
1               5                   10                  15

Gly Asn Leu Pro Asn Met Leu Arg Asp Leu Arg Asp Ala Phe Ser Arg
            20                  25                  30

Val Lys Thr Phe Phe Gln Met Lys Asp Gln Leu Asp Asn Leu Leu Leu
        35                  40                  45

Lys Glu Ser Leu Leu Glu Asp Phe Lys Gly Tyr Leu Gly Cys Gln Ala
    50                  55                  60

Leu Ser Glu Met Ile Gln Phe Tyr Leu Glu Glu Val Met Pro Gln Ala
65                  70                  75                  80

Glu Asn Gln Asp Pro Asp Ile Lys Ala His Val Asn Ser Leu Gly Glu
                85                  90                  95

Asn Leu Lys Thr Leu Arg Leu Arg Leu Arg Arg Cys His Arg Phe Leu
            100                 105                 110

Pro Cys Glu Asn Lys Ser Lys Ala Val Glu Gln Val Lys Asn Ala Phe
        115                 120                 125

Asn Lys Leu Gln Glu Lys Gly Ile Tyr Lys Ala Met Ser Glu Phe Asp
    130                 135                 140

Ile Phe Ile Asn Tyr Ile Glu Ala Tyr Met Thr Met Lys Ile Arg Asn
145                 150                 155                 160

<210> 2
<211> 160
<212> PRT
<213> mus musculus

<400> 2

Ser Arg Gly Gln Tyr Ser Arg Glu Asp Asn Asn Cys Thr His Phe Pro
1               5                   10                  15

Val Gly Gln Ser His Met Leu Leu Glu Leu Arg Thr Ala Phe Ser Gln
            20                  25                  30

Val Lys Thr Phe Phe Gln Thr Lys Asp Gln Leu Asp Asn Ile Leu Leu
        35                  40                  45

Thr Asp Ser Leu Met Gln Asp Phe Lys Gly Tyr Leu Gly Cys Gln Ala
    50                  55                  60

Leu Ser Glu Met Ile Gln Phe Tyr Leu Val Glu Val Met Pro Gln Ala
65                  70                  75                  80

Glu Lys His Gly Pro Glu Ile Lys Glu His Leu Asn Ser Leu Gly Glu
                85                  90                  95

Lys Leu Lys Thr Leu Arg Met Arg Leu Arg Arg Cys His Arg Phe Leu
            100                 105                 110

Pro Cys Glu Asn Lys Ser Lys Ala Val Glu Gln Val Lys Ser Asp Phe
        115                 120                 125

Asn Lys Leu Gln Asp Gln Gly Val Tyr Lys Ala Met Asn Glu Phe Asp
    130                 135                 140

Ile Phe Ile Asn Cys Ile Glu Ala Tyr Met Met Ile Lys Met Lys Ser
145                 150                 155                 160

<210>  3
<211>  160
<212>  PRT
<213>  macaca mulatta

<400>  3

Ser Pro Gly Gln Gly Thr Gln Ser Glu Asn Ser Cys Thr Arg Phe Pro
1               5                   10                  15

Gly Asn Leu Pro His Met Leu Arg Asp Leu Arg Asp Ala Phe Ser Arg
            20                  25                  30

Val Lys Thr Phe Phe Gln Met Lys Asp Gln Leu Asp Asn Ile Leu Leu
        35                  40                  45

Lys Glu Ser Leu Leu Glu Asp Phe Lys Gly Tyr Leu Gly Cys Gln Ala
    50                  55                  60

Leu Ser Glu Met Ile Gln Phe Tyr Leu Glu Glu Val Met Pro Gln Ala
65                  70                  75                  80

Glu Asn His Asp Pro Asp Ile Lys Glu His Val Asn Ser Leu Gly Glu
                85                  90                  95

Asn Leu Lys Thr Leu Arg Leu Arg Leu Arg Arg Cys His Arg Phe Leu
            100                 105                 110

Pro Cys Glu Asn Lys Ser Lys Ala Val Glu Gln Val Lys Asn Ala Phe
        115                 120                 125

Ser Lys Leu Gln Glu Lys Gly Val Tyr Lys Ala Met Ser Glu Phe Asp
    130                 135                 140

Ile Phe Ile Asn Tyr Ile Glu Ala Tyr Met Thr Met Lys Ile Gln Asn
145                 150                 155                 160

<210>  4
<211>  172
<212>  PRT
<213>  Canis lupus familiaris

<400>  4

Met His Gly Ser Ala Leu Leu Cys Cys Cys Leu Val Leu Leu Ala Gly
1               5                   10                  15

Val Gly Ala Ser Arg His Gln Ser Thr Leu Leu Glu Asp Asp Pro His
            20                  25                  30

Met Leu Arg Glu Leu Arg Ala Ala Phe Gly Arg Val Lys Ile Phe Phe
        35                  40                  45

Gln Met Lys Asp Lys Leu Asp Asn Ile Leu Leu Thr Gly Ser Leu Leu
    50                  55                  60

Glu Asp Phe Lys Ser Tyr Leu Gly Cys Gln Ala Leu Ser Glu Met Ile
65                  70                  75                  80

Gln Phe Tyr Leu Glu Glu Val Met Pro Arg Ala Glu Asn His Asp Pro
                85                  90                  95

Asp Ile Lys Asn His Val Asn Ser Leu Gly Glu Lys Leu Lys Thr Leu
            100                 105                 110

Arg Leu Arg Leu Arg Leu Arg Arg Cys His Arg Phe Leu Pro Cys Glu
        115                 120                 125

Asn Lys Ser Lys Ala Val Glu Gln Val Lys Ser Ala Phe Ser Lys Leu
    130                 135                 140

Gln Glu Lys Gly Val Tyr Lys Ala Met Ser Glu Phe Asp Ile Phe Ile
145                 150                 155                 160

Asn Tyr Ile Glu Thr Tyr Met Thr Met Arg Met Lys
                165                 170

<210>  5
<211>  178
<212>  PRT
<213>  rattus norvegicus

<400>  5

Met Pro Gly Ser Ala Leu Leu Cys Cys Leu Leu Leu Leu Ala Gly Val
1               5                   10                  15

Lys Thr Ser Lys Gly His Ser Ile Arg Gly Asp Asn Asn Cys Thr His
            20                  25                  30

Phe Pro Val Ser Gln Thr His Met Leu Arg Glu Leu Arg Ala Ala Phe
        35                  40                  45

Ser Gln Val Lys Thr Phe Phe Gln Lys Lys Asp Gln Leu Asp Asn Ile
    50                  55                  60

Leu Leu Thr Asp Ser Leu Leu Gln Asp Phe Lys Gly Tyr Leu Gly Cys
65                  70                  75                  80

Gln Ala Leu Ser Glu Met Ile Lys Phe Tyr Leu Val Glu Val Met Pro
                85                  90                  95

Gln Ala Glu Asn His Gly Pro Glu Ile Lys Glu His Leu Asn Ser Leu
            100                 105                 110

Gly Glu Lys Leu Lys Thr Leu Trp Ile Gln Leu Arg Arg Cys His Arg
        115                 120                 125

Phe Leu Pro Cys Glu Asn Lys Ser Lys Ala Val Glu Gln Val Lys Asn
    130                 135                 140

Asp Phe Asn Lys Leu Gln Asp Lys Gly Val Tyr Lys Ala Met Asn Glu
145                 150                 155                 160

Phe Asp Ile Phe Ile Asn Cys Ile Glu Ala Tyr Val Thr Leu Lys Met
                165                 170                 175

Lys Asn

<210>  6
<211>  178
<212>  PRT
<213>  cavia porcellus

<400>  6

Met Pro Gly Ser Ala Leu Leu Cys Cys Leu Ala Leu Leu Ala Gly Val
1               5                   10                  15

Lys Ala Ser Gln Gly Thr Asn Thr Gln Ser Glu Asp Ser Cys Ala His
            20                  25                  30

Phe Pro Ala Gly Leu Pro His Met Leu Arg Glu Leu Arg Ala Ala Phe
        35                  40                  45

Gly Arg Val Lys Thr Phe Phe Gln Thr Gln Asp Gln Leu Asp Asn Val
    50                  55                  60

Leu Leu Asn Lys Ser Leu Leu Glu Asp Phe Lys Gly Tyr Leu Gly Cys
65                  70                  75                  80

Gln Ala Leu Ser Glu Met Ile Gln Phe Tyr Leu Val Glu Val Met Pro
                85                  90                  95

Gln Ala Glu Lys His Gly Pro Glu Ile Lys Glu His Leu Asn Ser Leu
            100                 105                 110

Gly Glu Lys Leu Lys Thr Leu Arg Met Arg Leu Arg Arg Cys His Arg
        115                 120                 125

Phe Leu Pro Cys Glu Asn Lys Ser Lys Ala Val Glu Gln Val Lys Ser
    130                 135                 140

Asp Phe Asn Lys Leu Gln Asp Gln Gly Val Tyr Lys Ala Met Asn Glu
145                 150                 155                 160

Phe Asp Ile Phe Ile Asn Cys Ile Glu Ala Tyr Met Met Ile Lys Met
                165                 170                 175

Lys Ser

<210>  7
<211>  175
<212>  PRT
<213>  sus scrofa

<400>  7

Met Pro Ser Ser Ala Leu Leu Tyr Cys Leu Ile Phe Leu Ala Gly Val
1               5                   10                  15

Ala Ala Ser Ile Lys Ser Glu Asn Ser Cys Ile His Phe Pro Thr Ser
            20                  25                  30

Leu Pro His Met Leu Arg Glu Leu Arg Ala Ala Phe Gly Pro Val Lys
        35                  40                  45

Ser Phe Phe Gln Thr Lys Asp Gln Met Gly Asp Leu Leu Leu Thr Gly
    50                  55                  60

Ser Leu Leu Glu Asp Phe Lys Gly Tyr Leu Gly Cys Gln Ala Leu Ser
65                  70                  75                  80

Glu Met Ile Gln Phe Tyr Leu Glu Asp Val Met Pro Lys Ala Glu Ser
                85                  90                  95

Asp Gly Glu Asp Ile Lys Glu His Val Asn Ser Leu Gly Glu Lys Leu
            100                 105                 110

Lys Thr Leu Arg Leu Arg Leu Arg Arg Cys His Gln Phe Leu Pro Cys
        115                 120                 125

Glu Asn Lys Ser Lys Ala Val Glu Glu Val Lys Ser Ala Phe Ser Lys
    130                 135                 140

Leu Gln Glu Arg Gly Val Tyr Lys Ala Met Gly Glu Phe Asp Ile Phe
145                 150                 155                 160

Ile Asn Tyr Ile Glu Ala Tyr Met Thr Met Lys Met Arg Lys Asn
                165                 170                 175

<210>  8
<211>  178
<212>  PRT
<213>  homo sapiens

<400>  8

Met His Ser Ser Ala Leu Leu Cys Cys Leu Val Leu Leu Thr Gly Val
1               5                   10                  15

Arg Ala Ser Pro Gly Gln Gly Thr Gln Ser Glu Asn Ser Cys Thr His
            20                  25                  30

Phe Pro Gly Asn Leu Pro Asn Met Leu Arg Asp Leu Arg Asp Ala Phe
        35                  40                  45

Ser Arg Val Lys Thr Phe Phe Gln Met Lys Asp Gln Leu Asp Asn Leu
    50                  55                  60

Leu Leu Lys Glu Ser Leu Leu Glu Asp Phe Lys Gly Tyr Leu Gly Cys
65                  70                  75                  80

Gln Ala Leu Ser Glu Met Ile Gln Phe Tyr Leu Glu Glu Val Met Pro
                85                  90                  95

Gln Ala Glu Asn Gln Asp Pro Asp Ile Lys Ala His Val Asn Ser Leu
            100                 105                 110

Gly Glu Asn Leu Lys Thr Leu Arg Leu Arg Leu Arg Arg Cys His Arg
        115                 120                 125

Phe Leu Pro Cys Glu Asn Lys Ser Lys Ala Val Glu Gln Val Lys Asn
    130                 135                 140

Ala Phe Asn Lys Leu Gln Glu Lys Gly Ile Tyr Lys Ala Met Ser Glu
145                 150                 155                 160

Phe Asp Ile Phe Ile Asn Tyr Ile Glu Ala Tyr Met Thr Met Lys Ile
                165                 170                 175

Arg Asn

<210>  9
<211>  178
<212>  PRT
<213>  mus musculus

<400>  9

Met Pro Gly Ser Ala Leu Leu Cys Cys Leu Leu Leu Leu Thr Gly Met
1               5                   10                  15

Arg Ile Ser Arg Gly Gln Tyr Ser Arg Glu Asp Asn Asn Cys Thr His
            20                  25                  30

Phe Pro Val Gly Gln Ser His Met Leu Leu Glu Leu Arg Thr Ala Phe
        35                  40                  45

Ser Gln Val Lys Thr Phe Phe Gln Thr Lys Asp Gln Leu Asp Asn Ile
    50                  55                  60

Leu Leu Thr Asp Ser Leu Met Gln Asp Phe Lys Gly Tyr Leu Gly Cys
65                  70                  75                  80

Gln Ala Leu Ser Glu Met Ile Gln Phe Tyr Leu Val Glu Val Met Pro
                85                  90                  95

Gln Ala Glu Lys His Gly Pro Glu Ile Lys Glu His Leu Asn Ser Leu
            100                 105                 110

Gly Glu Lys Leu Lys Thr Leu Arg Met Arg Leu Arg Arg Cys His Arg
        115                 120                 125

Phe Leu Pro Cys Glu Asn Lys Ser Lys Ala Val Glu Gln Val Lys Ser
    130                 135                 140

Asp Phe Asn Lys Leu Gln Asp Gln Gly Val Tyr Lys Ala Met Asn Glu
145                 150                 155                 160

Phe Asp Ile Phe Ile Asn Cys Ile Glu Ala Tyr Met Met Ile Lys Met
                165                 170                 175

Lys Ser

<210>  10

<211>  178
<212>  PRT
<213>  macaca mulatta

<400>  10

Met His Ser Ser Ala Leu Leu Cys Cys Leu Val Leu Leu Thr Gly Val
1               5                   10                  15

Arg Ala Ser Pro Gly Gln Gly Thr Gln Ser Glu Asn Ser Cys Thr Arg
            20                  25                  30

Phe Pro Gly Asn Leu Pro His Met Leu Arg Asp Leu Arg Asp Ala Phe
        35                  40                  45

Ser Arg Val Lys Thr Phe Phe Gln Met Lys Asp Gln Leu Asp Asn Ile
    50                  55                  60

Leu Leu Lys Glu Ser Leu Leu Glu Asp Phe Lys Gly Tyr Leu Gly Cys
65                  70                  75                  80

Gln Ala Leu Ser Glu Met Ile Gln Phe Tyr Leu Glu Glu Val Met Pro
                85                  90                  95

Gln Ala Glu Asn His Asp Pro Asp Ile Lys Glu His Val Asn Ser Leu
            100                 105                 110

Gly Glu Asn Leu Lys Thr Leu Arg Leu Arg Leu Arg Arg Cys His Arg
        115                 120                 125

Phe Leu Pro Cys Glu Asn Lys Ser Lys Ala Val Glu Gln Val Lys Asn
    130                 135                 140

Ala Phe Ser Lys Leu Gln Glu Lys Gly Val Tyr Lys Ala Met Ser Glu
145                 150                 155                 160

Phe Asp Ile Phe Ile Asn Tyr Ile Glu Ala Tyr Met Thr Met Lys Ile
                165                 170                 175

Gln Asn

<210>  11
<211>  134
<212>  PRT
<213>  homo sapiens

<400>  11

Met Arg Met Leu Leu His Leu Ser Leu Leu Ala Leu Gly Ala Ala Tyr
1               5                   10                  15

Val Tyr Ala Ile Pro Thr Glu Ile Pro Thr Ser Ala Leu Val Lys Glu
            20                  25                  30

Thr Leu Ala Leu Leu Ser Thr His Arg Thr Leu Leu Ile Ala Asn Glu
        35                  40                  45

Thr Leu Arg Ile Pro Val Pro Val His Lys Asn His Gln Leu Cys Thr
    50                  55                  60

Glu Glu Ile Phe Gln Gly Ile Gly Thr Leu Glu Ser Gln Thr Val Gln
65                  70                  75                  80

Gly Gly Thr Val Glu Arg Leu Phe Lys Asn Leu Ser Leu Ile Lys Lys
                85                  90                  95

Tyr Ile Asp Gly Gln Lys Lys Lys Cys Gly Glu Glu Arg Arg Arg Val
            100                 105                 110

Asn Gln Phe Leu Asp Tyr Leu Gln Glu Phe Leu Gly Val Met Asn Thr
        115                 120                 125

Glu Trp Ile Ile Glu Ser
    130

<210>  12
<211>  133
<212>  PRT
<213>  mus musculus

<400>  12

Met Arg Arg Met Leu Leu His Leu Ser Val Leu Thr Leu Ser Cys Val
1               5                   10                  15

Trp Ala Thr Ala Met Glu Ile Pro Met Ser Thr Val Val Lys Glu Thr
            20                  25                  30

Leu Thr Gln Leu Ser Ala His Arg Ala Leu Leu Thr Ser Asn Glu Thr
        35                  40                  45

Met Arg Leu Pro Val Pro Thr His Lys Asn His Gln Leu Cys Ile Gly
    50                  55                  60

Glu Ile Phe Gln Gly Leu Asp Ile Leu Lys Asn Gln Thr Val Arg Gly
65                  70                  75                  80

Gly Thr Val Glu Met Leu Phe Gln Asn Leu Ser Leu Ile Lys Lys Tyr
                85                  90                  95

Ile Asp Arg Gln Lys Glu Lys Cys Gly Glu Glu Arg Arg Arg Thr Arg
            100                 105                 110

Gln Phe Leu Asp Tyr Leu Gln Glu Phe Leu Gly Val Met Ser Thr Glu
        115                 120                 125

Trp Ala Met Glu Gly
    130

<210>  13
<211>  166
<212>  PRT
<213>  homo sapiens

<400>  13

Met Lys Tyr Thr Ser Tyr Ile Leu Ala Phe Gln Leu Cys Ile Val Leu
1               5                   10                  15

Gly Ser Leu Gly Cys Tyr Cys Gln Asp Pro Tyr Val Lys Glu Ala Glu
            20                  25                  30

Asn Leu Lys Lys Tyr Phe Asn Ala Gly His Ser Asp Val Ala Asp Asn
        35                  40                  45

Gly Thr Leu Phe Leu Gly Ile Leu Lys Asn Trp Lys Glu Glu Ser Asp
    50                  55                  60

Arg Lys Ile Met Gln Ser Gln Ile Val Ser Phe Tyr Phe Lys Leu Phe
65                  70                  75                  80

Lys Asn Phe Lys Asp Asp Gln Ser Ile Gln Lys Ser Val Glu Thr Ile
                85                  90                  95

Lys Glu Asp Met Asn Val Lys Phe Phe Asn Ser Asn Lys Lys Lys Arg
            100                 105                 110

Asp Asp Phe Glu Lys Leu Thr Asn Tyr Ser Val Thr Asp Leu Asn Val
        115                 120                 125

Gln Arg Lys Ala Ile His Glu Leu Ile Gln Val Met Ala Glu Leu Ser
    130                 135                 140

Pro Ala Ala Lys Thr Gly Lys Arg Lys Arg Ser Gln Met Leu Phe Arg
145                 150                 155                 160

Gly Arg Arg Ala Ser Gln
                165

<210>  14
<211>  155
<212>  PRT
<213>  mus musculus

<400>  14

Met Asn Ala Thr His Cys Ile Leu Ala Leu Gln Leu Phe Leu Met Ala
1               5                   10                  15

Val Ser Gly Cys Tyr Cys His Gly Thr Val Ile Glu Ser Leu Glu Ser
            20                  25                  30

Leu Asn Asn Tyr Phe Asn Ser Ser Gly Ile Asp Val Glu Glu Lys Ser
        35                  40                  45

Leu Phe Leu Asp Ile Trp Arg Asn Trp Gln Lys Asp Gly Asp Met Lys
    50                  55                  60

Ile Leu Gln Ser Gln Ile Ile Ser Phe Tyr Leu Arg Leu Phe Glu Val
65                  70                  75                  80

Leu Lys Asp Asn Gln Ala Ile Ser Asn Asn Ile Ser Val Ile Glu Ser
                85                  90                  95

His Leu Ile Thr Thr Phe Phe Ser Asn Ser Lys Ala Lys Lys Asp Ala
            100                 105                 110

Phe Met Ser Ile Ala Lys Phe Glu Val Asn Asn Pro Gln Val Gln Arg
        115                 120                 125

Gln Ala Phe Asn Glu Leu Ile Arg Val Val His Gln Leu Leu Pro Glu
    130                 135                 140

Ser Ser Leu Arg Lys Arg Lys Arg Ser Arg Cys
145                 150                 155

<210>  15
<211>  212
<212>  PRT
<213>  homo sapiens

<400>  15

Met Asn Ser Phe Ser Thr Ser Ala Phe Gly Pro Val Ala Phe Ser Leu
1               5                   10                  15

Gly Leu Leu Leu Val Leu Pro Ala Ala Phe Pro Ala Pro Val Pro Pro
            20                  25                  30

Gly Glu Asp Ser Lys Asp Val Ala Ala Pro His Arg Gln Pro Leu Thr
        35                  40                  45

Ser Ser Glu Arg Ile Asp Lys Gln Ile Arg Tyr Ile Leu Asp Gly Ile
    50                  55                  60

Ser Ala Leu Arg Lys Glu Thr Cys Asn Lys Ser Asn Met Cys Glu Ser
65                  70                  75                  80

Ser Lys Glu Ala Leu Ala Glu Asn Asn Leu Asn Leu Pro Lys Met Ala
                85                  90                  95

Glu Lys Asp Gly Cys Phe Gln Ser Gly Phe Asn Glu Glu Thr Cys Leu
            100                 105                 110

Val Lys Ile Ile Thr Gly Leu Leu Glu Phe Glu Val Tyr Leu Glu Tyr
        115                 120                 125

Leu Gln Asn Arg Phe Glu Ser Ser Glu Glu Gln Ala Arg Ala Val Gln
    130                 135                 140

Met Ser Thr Lys Val Leu Ile Gln Phe Leu Gln Lys Lys Ala Lys Asn
145                 150                 155                 160

Leu Asp Ala Ile Thr Thr Pro Asp Pro Thr Thr Asn Ala Ser Leu Leu
                165                 170                 175

Thr Lys Leu Gln Ala Gln Asn Gln Trp Leu Gln Asp Met Thr Thr His
            180                 185                 190

Leu Ile Leu Arg Ser Phe Lys Glu Phe Leu Gln Ser Ser Leu Arg Ala
        195                 200                 205

Leu Arg Gln Met
    210

<210>  16
<211>  211
<212>  PRT
<213>  mus musculus

<400>  16

Met Lys Phe Leu Ser Ala Arg Asp Phe His Pro Val Ala Phe Leu Gly
1               5                   10                  15

Leu Met Leu Val Thr Thr Thr Ala Phe Pro Thr Ser Gln Val Arg Arg
            20                  25                  30

Gly Asp Phe Thr Glu Asp Thr Thr Pro Asn Arg Pro Val Tyr Thr Thr
        35                  40                  45

Ser Gln Val Gly Gly Leu Ile Thr His Val Leu Trp Glu Ile Val Glu
    50                  55                  60

Met Arg Lys Glu Leu Cys Asn Gly Asn Ser Asp Cys Met Asn Asn Asp
65                  70                  75                  80

Asp Ala Leu Ala Glu Asn Asn Leu Lys Leu Pro Glu Ile Gln Arg Asn
                85                  90                  95

Asp Gly Cys Tyr Gln Thr Gly Tyr Asn Gln Glu Ile Cys Leu Leu Lys
            100                 105                 110

Ile Ser Ser Gly Leu Leu Glu Tyr His Ser Tyr Leu Glu Tyr Met Lys
        115                 120                 125

Asn Asn Leu Lys Asp Asn Lys Lys Asp Lys Ala Arg Val Leu Gln Arg
    130                 135                 140

Asp Thr Glu Thr Leu Ile His Ile Phe Asn Gln Glu Val Lys Asp Leu
145                 150                 155                 160

His Lys Ile Val Leu Pro Thr Pro Ile Ser Asn Ala Leu Leu Thr Asp
                165                 170                 175

Lys Leu Glu Ser Gln Lys Glu Trp Leu Arg Thr Lys Thr Ile Gln Phe
            180                 185                 190

Ile Leu Lys Ser Leu Glu Glu Phe Leu Lys Val Thr Leu Arg Ser Thr
        195                 200                 205

Arg Gln Thr
    210